# EUROPEAN PATENT SPECIFICATION

(11) **EP 2 645 936 B1**
(45) Date of publication and mention of the grant of the patent: **07.09.2016**
(21) Application number: 11794349.8
(22) Date of filing: 30.11.2011
(51) Int. Cl.: A61B 6/10, G21F 1/12, G21F 3/00, A61B 6/00, G21F 1/00

(54) **RADIATION SHIELD ASSEMBLY AND METHOD OF PROVIDING A STERILE BARRIER TO RADIATION**
STRAHLUNGSSCHUTZANORDNUNG UND VERFAHREN ZUR BEREITSTELLUNG EINER STERILEN BARRIERE GEGENÜNER STRAHLUNG
ENSEMBLE DE PROTECTION CONTRE LES RAYONNEMENTS ET PROCÉDÉ DE RÉALISATION D'UNE BARRIÈRE STÉRILE CONTRE LES RAYONNEMENTS

(30) Priority: 30.11.2010 US 418328 P
(43) Date of publication of application: 09.10.2013
(73) Proprietor: Interventco, LLC, Dallas, TX 75225 (US)
(72) Inventor: REES, Chet, R., Dallas, TX 75225 (US)
(74) Representative: Gosnall, Toby
(86) International application number: PCT/US2011/062630
(87) International publication number: WO 2012/121765

(56) References cited:
- WO-A1-01/26551
- WO-A2-2007/060561
- WO-A2-2008/086338
- DE-U1- 29 706 321
- US-A1- 2006 124 871
- US-A1- 2006 156 472
- US-A1- 2006 251 219
- US-A1- 2007 029 513
- US-A1- 2008 035 159

## Description

### BACKGROUND OF THE INVENTION

### 1. Technical Field

This invention relates generally to radiation shields used in surgical procedures, and more particularly to reusable radiation shield assemblies and method of providing a sterile barrier to radiation.

### 2. Related Art

Many medical and veterinary procedures involve the use of X-ray radiation while an operator/surgeon is manipulating instruments. Often, there is a sterile field in which the operator works, which includes at least a portion of the patient or subject. The radiation must pass through the patient in order to create the images, but scatter radiation is inevitably produced, which passes in many directions and does not contribute to the desired goal of imaging, and can result in harm to the patient, operator, or others in the area.

Surgeons continue to strive to reduce their exposure to scatter radiation including various shields, shielding garments, and barriers. However, maintaining them in the path of the scatter radiation without obstructing work continues to prove difficult, as is the maintenance of sterility in the operative field/theatre while attempting to position radiation shielding.

One solution available commercially involves blankets or drapes containing radiation barrier materials such as bismuth, antimony, barium, lead, tin, nano-compounds, Demron™, and others. These may be laid on the patient in a manner that reduces scatter to the surgeon, but does not hinder the procedure. However, maintaining the known devices in their intended locations remains a challenge. Further, because such positioning usually involves placement in the sterile field, the devices are sterilized and packaged sterily for use. This causes the continued problem of their need to be disposable, resulting in creation of a large quantity of medical waste containing infectious bodily fluids, and the consumption of large amounts of the heavy metal materials for each disposable blanket. This involves depletion of these materials, and toxic material disposal issues. Because they are disposable, conservation of materials may mitigate towards the use of thinner shielding, for example, 0.25 mm Pb equivalency in some commercial products. A non- disposable alternative, as proposed in this invention, could be thicker, for example 0.5 mm Pb, in order to provide better protection, while using far less material due to its re-usable nature.

Here we propose an invention that can, among other things, provide the beneficial properties of the disposable radiation shielding blankets while at the same time reducing the consumption and disposal of toxic or non-toxic heavy metals or otherwise expensive materials, reduce the overall bulk of all disposable materials, maintaining the sterile field, and be reliably positioned and maintained in position, as desired, throughout the procedure without unwanted movement.

US 2006/124871 (A1) discloses a radiation protection arrangement for screening radiation emitted from a radiation source, especially an x-ray source. Said arrangement is provided with a screening element consisting of, or comprising, a radiation protection material, and a cover, which fully surrounds the screening element. Said cover can be pulled over the screening element and completely separated from the same. As the cover can be changed, the radiation protection arrangement can be kept clean and sterile in a simple manner.

### SUMMARY OF THE INVENTION

In accordance with one aspect of the invention, a radiation shield assembly according to claim 1 is provided.

The radiation shield assembly may further comprise any of the optional features described in claims 2 to 12.

In accordance with another aspect of the invention, there is provided the method of providing a sterile barrier to radiation in a surgical procedure according to claim 13.

The method of providing a sterile barrier to radiation in a surgical procedure may further comprise any of the optional features described in claims 14 to 15.

### BRIEF DESCRIPTION OF THE DRAWINGS

These and other aspects, features and advantages of the present invention will become more readily appreciated when considered in connection with the following detailed description of presently preferred embodiments and best mode, appended claims and accompanying drawings, in which:
Figure 1 is a sequential assembly of a radiation shield and drape assembly constructed in accordance with one aspect of the invention;
Figure 2 is a sequential assembly of a radiation shield and drape assembly constructed in accordance with another aspect of the invention;
Figures 3 and 3A show a sequential assembly of a radiation shield and drape assembly constructed in accordance with another aspect of the invention;
Figure 3B shows a fastener member constructed in accordance with another aspect of the invention for use with the assembly of Figure 3;
Figure 3C shows a radiation shield and drape assembly constructed in accordance with another aspect of the invention;
Figure 3D shows a radiation shield and drape assembly constructed in accordance with another aspect of the invention;
Figure 3E shows a radiation shield and drape assembly constructed in accordance with another aspect of the invention;
Figures 4 is an exploded view of a radiation shield and drape assembly constructed in accordance with another aspect of the invention;
Figures 4A and 4B show a radiation shield and drape assembly constructed in accordance with another aspect of the invention;
Figure 5 is an exploded view of a radiation shield and drape assembly constructed in accordance with another aspect of the invention;
Figure 5A is an assembled view of the radiation shield and drape assembly of Figure 5 including a fastener strap in accordance with another aspect of the invention;
Figure 6 is an exploded view of a radiation shield and drape assembly constructed in accordance with another aspect of the invention;
Figure 6A is an assembled view of the radiation shield and drape assembly of Figure 6;
Figure 7 is an exploded view of modular components of a radiation shield and drape assembly constructed in accordance with another aspect of the invention;
Figure 7A is an assembled view of the modular radiation shield and drape assembly of Figure 7 shown in use with a sterile drape;
Figure 8 is a plan view of a modular radiation shield and drape assembly constructed in accordance with another aspect of the invention;
Figure 9 is a plan view of a radiation shield and drape assembly constructed in accordance with another aspect of the invention;
Figures 10A-10C show a patient on a surgical table with a radiation shield and drape assembly constructed in accordance with another aspect of the invention being disposed over selected areas of the patient;
Figure 11 shows a radiation shield constructed in accordance with another aspect of the invention;
Figures 12 and 12A show a radiation shield constructed in accordance with another aspect of the invention;
Figure 13 shows a radiation shield constructed in accordance with another aspect of the invention;
Figure 13A shows a joint of the radiation shield of Figure 13;
Figures 13B shows a radiation shield constructed in accordance with another aspect of the invention;
Figure 13C shows a joint of the radiation shield of Figure 13B;
Figure 13D shows an alternate embodiment of a joint of the radiation shields of Figures 13 and 13B;
Figure 14 illustrates a radiation shield constructed in accordance with another aspect of the invention;
Figure 15 illustrates a radiation shield constructed in accordance with another aspect of the invention;
Figures 16 and 16A illustrate a radiation shield constructed in accordance with another aspect of the invention;
Figures 17-17C illustrate a sequence of applying a radiation shield and drape constructed in accordance with another aspect of the invention over a patient;
Figure 18 is an exploded view of a radiation shield shown partially constructed in accordance with another aspect of the invention;
Figures 18A is an assembled view of the partially constructed radiation shield of Figure 18;
Figure 18B is view of the radiation shield of Figure 18A shown in a completed state of construction;
Figures 19 and 19A are assembled views of a radiation shield constructed in accordance with another aspect of the invention;
Figures 20 and 20A illustrate a patient with a surgical site being covered by the radiation shield of Figure 19;
Figures 21 and 21A illustrate a perspective view of a radiation shield support member constructed in accordance with another aspect of the invention;
Figure 21B illustrates a perspective view of a radiation shield support member constructed in accordance with another aspect of the invention;
Figures 22 and 22A illustrate the radiation shield support member of Figures 21 and 21A being used in combination with a radiopaque shield and drape assembly in a surgical procedure;
Figure 22B illustrates the radiation shield support member of Figure 21B being used in combination with a radiopaque shield and drape assembly in a surgical procedure;
Figure 23 is a perspective view of a radiation shield support member in combination with a radiation shield and drape assembly in accordance with another aspect of the invention;
Figure 24 illustrates the radiation shield support member and radiation shield and drape assembly of Figure 23 being used in a surgical procedure;
Figures 25-25C illustrate a perspective view of a radiation shield support member and radiation shield and drape assembly constructed in accordance with another aspect of the invention;
Figures 26-26D illustrate a perspective view of a radiation shield support member and radiation shield and drape assembly constructed in accordance with another aspect of the invention;
Figure 26E illustrates a perspective view of a radiation shield support member and radiation shield and drape assembly constructed in accordance with another aspect of the invention;
Figures 27-27C illustrate a radiation shield support member and radiation shield and drape assembly constructed in accordance with another aspect of the invention;
Figures 28 and 28A illustrate a radiation shield support member constructed in accordance with another aspect of the invention; and
Figures 29-29C illustrate a radiation shield support member and radiation shield and drape assembly constructed in accordance with another aspect of the invention.

### DETAILED DESCRIPTION OF PRESENTLY PREFERRED EMBODIMENTS

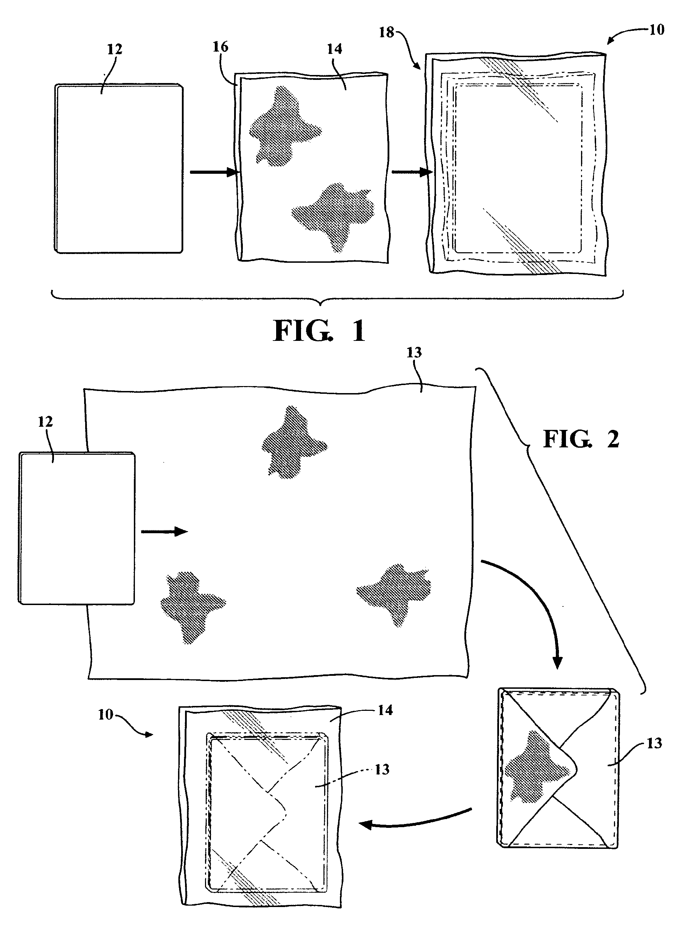

Referring in more detail to the drawings, Figure 1 illustrates an embodiment of a radiation shield and drape assembly 10 constructed in accordance with one aspect of the invention. The assembly 10 includes an insert 12 made of radiation shielding material, also referred to as radiopaque material. The insert 12 can be non-sterile and is generally provided to be re-usable. The insert 12 can be wrapped or contained inside a sterile drape 14 of a flexible material that is impervious to fluid. The drape 14 can be configured as a sterile plastic bag or pouch with an opening 16 that allows insertion of the insert 12 therein, wherein the opening 16 is closable in a air/fluid tight manner via a suitable fastener mechanism, referred to as fastener 18. Many types of fastening mechanisms could be used to form the fastener 18, including a simple folding flap to conceal the opening 16, a resealable type seal (e.g. adhesive or zip-type lock), heat-induced melt seal, or a clamp device that extends across the entire opening 16 and seals it closed. In addition, a sliding gripper may be used to slide over the opening and provide compressive sealing of the open surfaces. For added protection of sterility (maintenance of sterility of outer drape without passage of any non-sterile contaminants from the insert 12 to the external operative field/theater), as shown in Figure 2, the insert 12 may be first wrapped with redundancy in a larger sterile sheet 13, such as of plastic, paper, or other type of fabric, then it may be placed inside the sterile drape 14. Otherwise, the radiopaque insert material 12 may simply be wrapped in the sterile sheet 13, without being placed in a bag-shaped drape, with the sterile sheet 13 providing a sufficient barrier to transgression of sterile field.

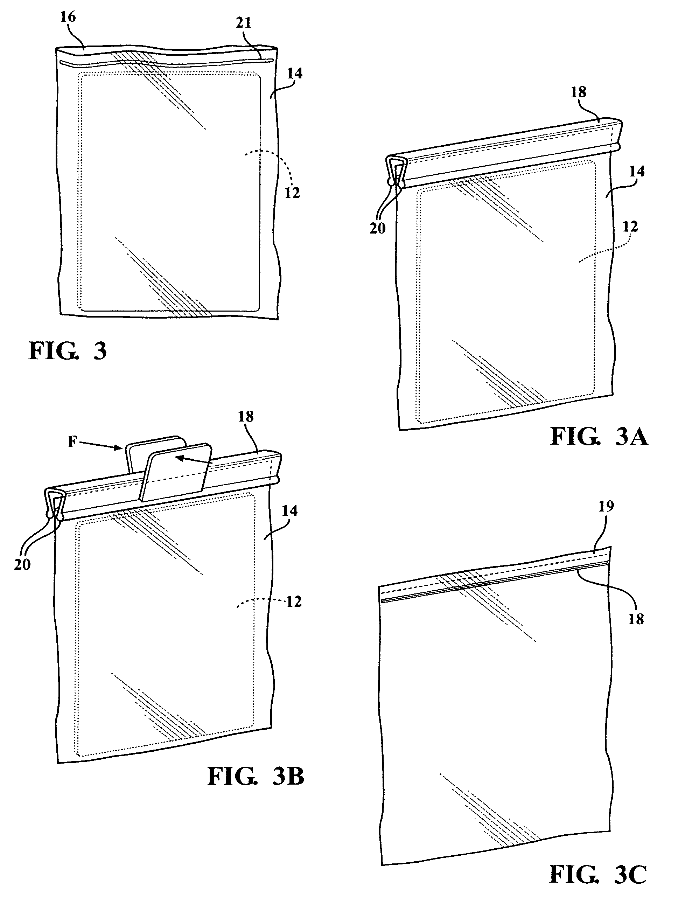

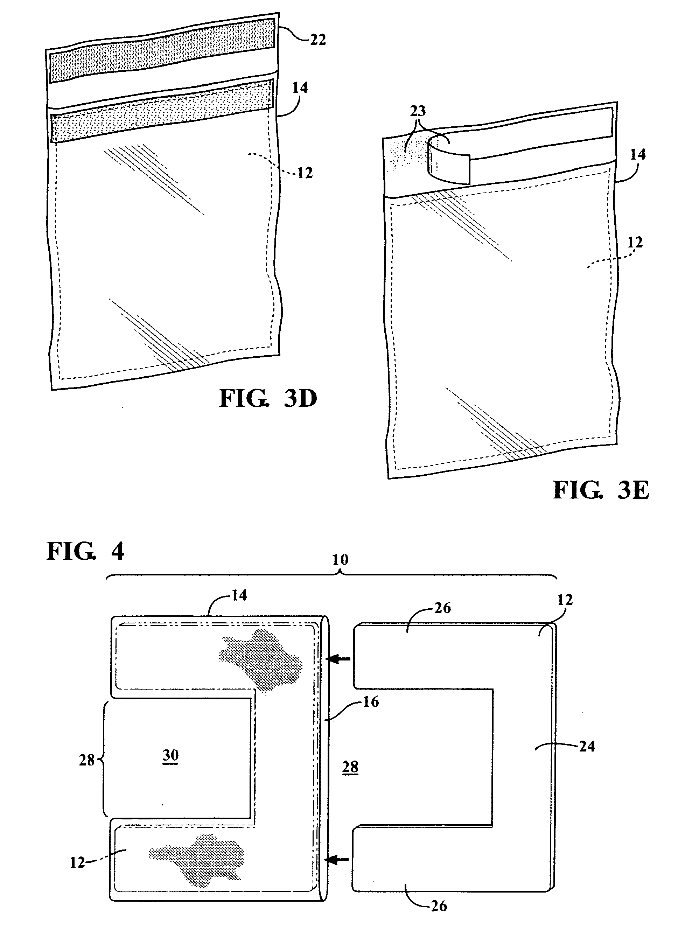

In Figure 3, an embodiment of a fastener 18 constructed in accordance with one aspect of the invention, also referred to as sealing mechanism or clamp, for the bag or drape 14 containing the sterile shielding insert 12, also referred to as shielding member or blanket is illustrated. The drape 14 includes an opening 16, shown as being located at one end. The insert 12 is inserted through the opening 16 and then the opening is sealed using the clamp 18 that slides on from one side of the drape 14 toward the opposite side. The clamp 18 is constructed from plastic or metal of other material with adequate force and shape memory to provide a clamp-like force across the edges of the drape 14 that compresses the opposing walls of the drape 14 together for an air/fluid tight seal. To facilitate sliding the clamp 18 on the drape 12, curved edges 20 can be provided at one or both ends to keep the clamp 18 from digging into or binding against the opposing walls of the drape 14 as the clamp 18 is slid along the drape material. The edges 20 can be configured to provide increased surface area contact with the walls of the drape 14 to increase the surface area of the sealed region. Further yet, the drape 14 can have an elongate ridge or rib 21 adjacent the edge bounding the opening 16 to facilitate passage or sliding of the clamp 18, as well as inhibiting inadvertent dislodgement or removal of the clamp 18 once slid into position. The rib(s) 21, in addition to or in lieu of being on the drape 14, could be provided adjacent edges of the pinchers of the clamp 18, thereby further inhibiting inadvertent pulling of the clamp 18 off the drape 14. Embodiments using other closure mechanisms are contemplated herein, including a clip 18, such as shown in Figure 3B, that opens under an external bias force (F) and closes into abutment with the drape 14 under a spring force imparted by the clip 18. Further yet, as shown in Figure 3C, the drape 14 could have a resealable style fastener 18, such as that used to seal sandwich bags, for example, with an upper section being removable via a tear-away perforated tab 19, when desired, to gain access to the re-sealable opening in the sterile drape 14. In yet another embodiment, as shown in Figure 3D, the drape 14 could have an extended flap 22 capable of folding over after disposing the insert 12 into a pocket of the drape, and then fastened to an opposite side of the drape 14, such as via hook and loop type fastener (e.g. Velcro TM), or a strip of sterile tape that extends along the interface of the edge of the extended flap (22) with the body of the drape (14), or, as shown in Figure 3E, via a self adhesive and release paper 23, or some other type of fastener, such as glue.

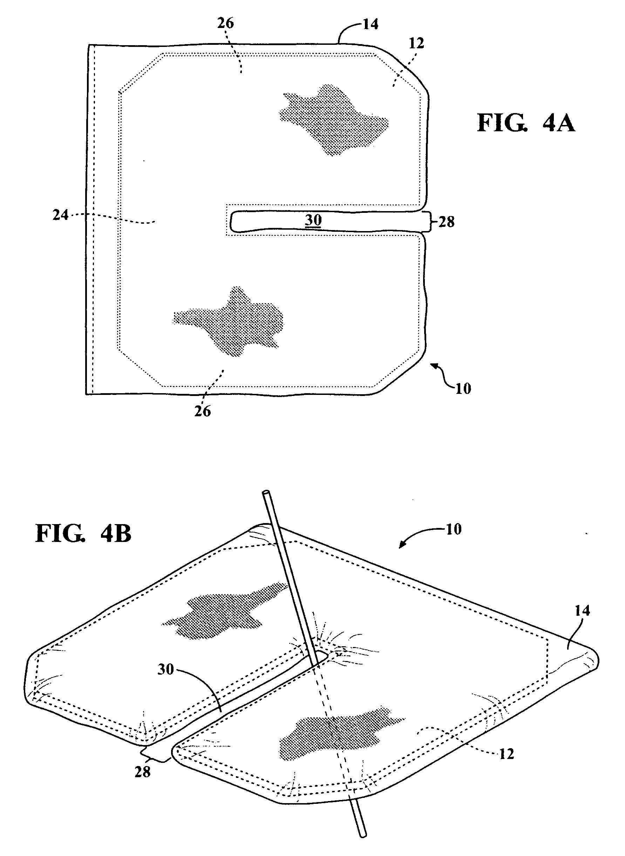

As shown in Figure 4, in accordance with another aspect of the invention, a non-sterile radiopaque shielding insert 12 is generally U-shaped having central leg 24 with a pair of side arms 26 extending a right angle from the leg 24 to form an open side 28. It should be recognized that instead of being U-shaped, the insert 12 could be generally C-shaped or otherwise configured having an open side, such as by being generally L-shaped by removal of one of the arms 26 of the generally U-shaped configuration. In the U-shaped embodiment, the arms 26 of the insert 12 are inserted through an opening 16 of a drape 14, wherein the drape 14 is configured having a slightly enlarged C-shape corresponding to the shape of the insert 12. Then, upon the arms 26 and leg 24 being received in the drape 14, the opening 16 is sealed closed using any type of desired closure mechanism, including those discussed in detail above. This U-shaped assembly 10 is functional in ways that a closed square or rectangular or other closed shape may not be, e.g., for placement around a site where an incision or other access to patient may be required. Thus, direct and scatter radiation is blocked by the assembly 10 while at the same time the surgeon's hands have access to the patient via a central opening 30 and open side 28 (referred to together as "open area"). The assembly 10 may be placed directly on the scrubbed patient's skin without contamination, and the surgeon may perform manipulations inside the open area. The open area could be made much smaller, to provide a smaller central opening area 30, shown in Figure 4A as being narrowed, but with wider shielding along the leg 24 and arms 26 to block more radiation scatter and reduce the risk of the surgeon being exposed to radiation during surgery, such as shown in Figure 4B.

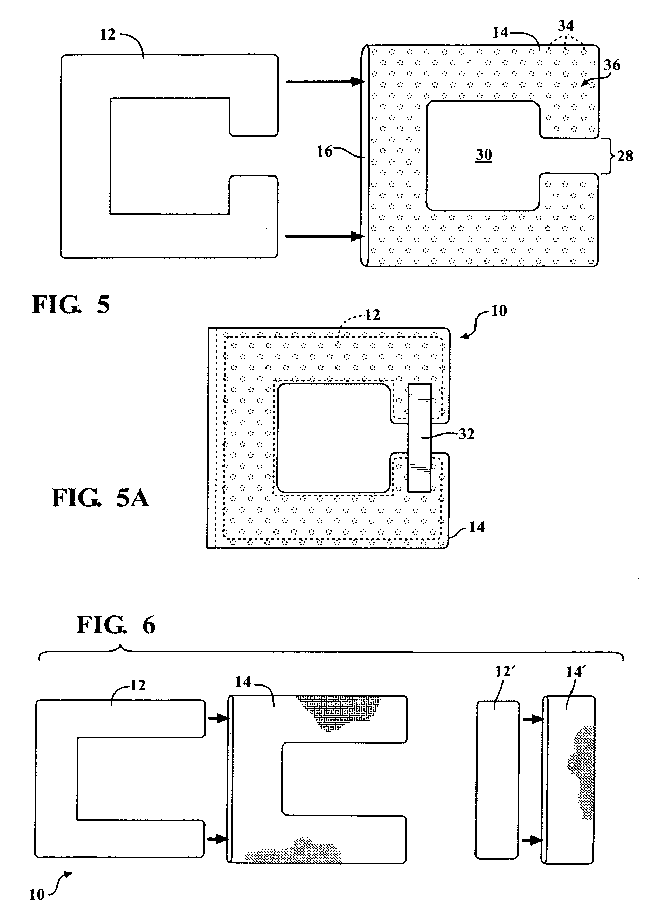

As shown in Figure 5, in accordance with another aspect of the invention, an insert 12 and corresponding drape 14 are each configured having a nearly or substantially closed circumference, but having a discontinuous circumferential perimeter, including a slit to provide an open side 28 along one side, and a central opening 30 in the substantially enclosed center. Again, the open area may be important for providing access to sterile working area on the patient, without transgressing the drape 14, thus maintaining sterility of the outer surfaces. Since the radiopaque insert 12 is flexible, it may be deformed somewhat as it is disposed through the opening 16 and inside the drape 14. Once the insert 12 is fully inserted, it can be unfolded to resume its fully unfolded natural shape to provide the radiation shielding function desired. Upon sealing the opening 16 of the drape 14, a fastener, such as a piece of sterile adhesive tape 32 (Figure 5A), or any type of fastener, e.g. hook and loop, or others discussed above, could then be used to bridge the open side 28, thereby fastening the two loose ends together to fully enclose the periphery of the assembly 10, thereby providing it with a circumferentially continuous outer periphery, and prevent the assembly from inadvertently changing shape considerably as it is manipulated. In addition, to facilitate maintaining the assembly 10 in its desired location in use, a gripping friction material, shown as a plurality of non-slip friction pads 34, such as discrete rubber nubs or some other high friction material, or adhesive tape strips, or strips of hook and loop fasteners that mate with the other sterile drapes in the operative field, can be fixed to a substantially at least one of the drapes opposite planar faces 36.

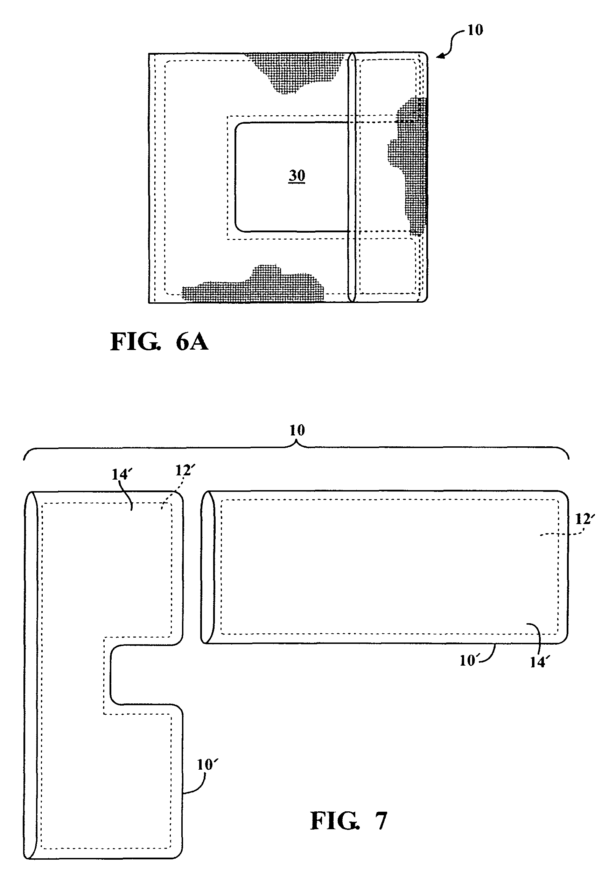

As shown in Figure 6, a radiation shield assembly 10 constructed in accordance with another aspect of the invention is illustrated. The assembly 10 includes a generally U-shaped radiopaque insert 12 enclosed in a sterile drape 14, such as described above with regard to Figure 4. Further, the assembly 10 includes a bridging rectangular insert 12' which is also enclosed in a separate sterile drape 14'. The two subassemblies may then be used in combination to create a shape having a closed, circumferentially continuous outer perimeter and a central opening 30 (Figure 6A). As such, the central opening 30 allows the surgeon access of the to a sterile field or patient skin through the central opening 30 while at the same time the circumferentially continuous boundary of radiopaque material provides a shield against exposure to scatter radiation to the surgeon's hands. The open space 30 may be much smaller, to provide more protective radiation shielding around it. The two modular components may be laid one on top of the other, and could be fastened together to prevent slipping using adhesive tape, hook and loop fasteners, or other fastening mechanism, such as those discussed above.

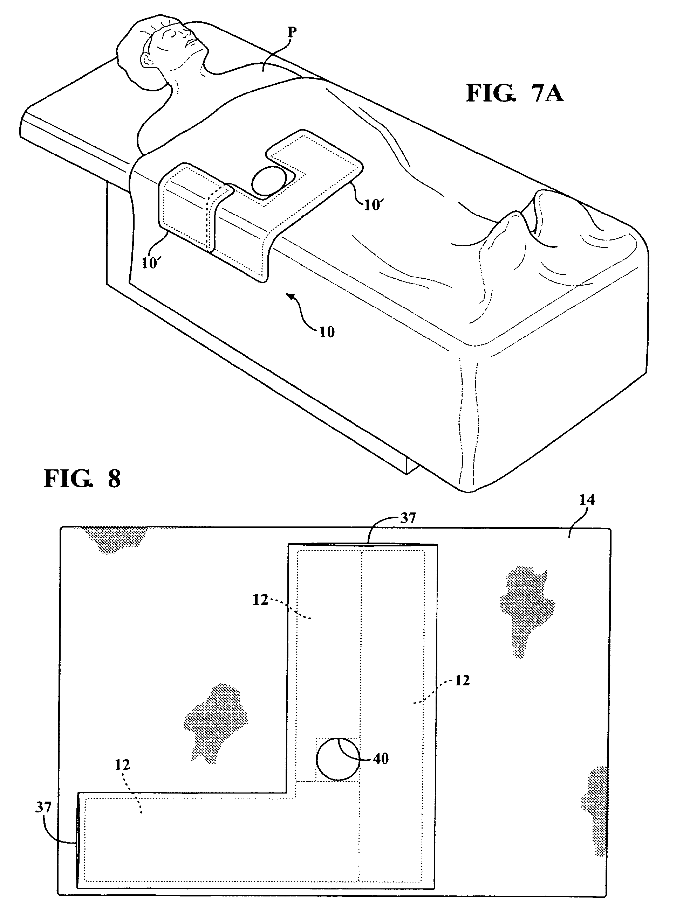

As shown in Figure 7A, a radiation shield assembly 10 constructed in accordance with another aspect of the invention is illustrated. In the disclosed embodiments, separate modular subassembly components 10' are shaped as shown. Each of the separate subassembly components 10' include a radiopaque shield insert 12' and a sterile drape 14' sized for receipt of the respective inserts 12' therein. The separate subassembly components 10' are configured to be overlapped slightly, as shown in Figure 7A. In the embodiment shown, the complete assembly forms a generally L-shaped configuration, which creates a working area surrounded partially on three sides, with a long extension on one side. This arrangement could be useful, for example, for a trans-femoral endovascular procedure with the surgeon standing on the right side of the patient P, so the radiation scatter is blocked towards the patient's feet and right side, where scatter would be particularly abundantly transmitted to surgeon in this situation. The radiation shield assembly 10 may be manipulated to hang to patient's right so as not to interfere with the direct beam of radiation over patient's abdomen. If the tube angle of the fluoroscopy unit is changed, the subassemblies 10' of shield 10 in the direct beam could easily be removed or manipulated by the surgeon, particularly since it is sterile, due to the outer sterile drape 14'. This could leave the other subassembly 10' of shield assembly in place, providing partial protection and not interfering with imaging over patient's torso. When the shapes of the subassemblies 10' are asymmetrical, they may be flipped over for use on the other side of the patient P.

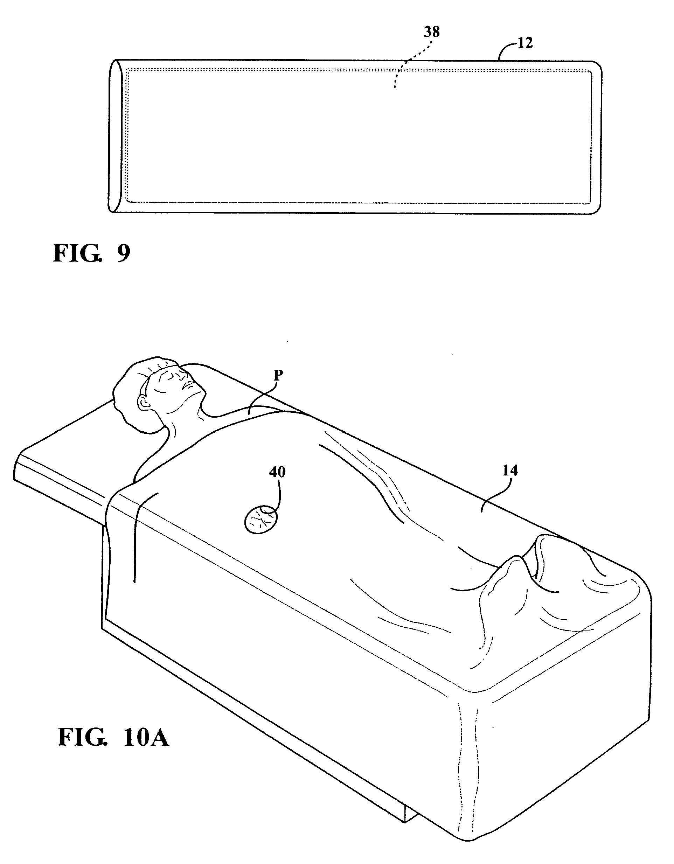

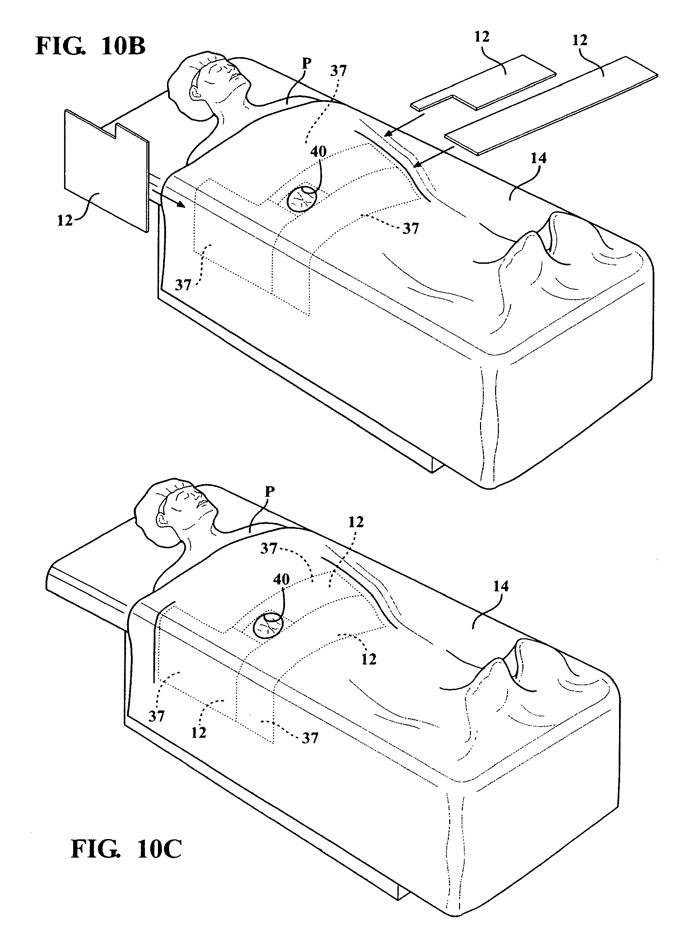

In accordance with another aspect of the invention, as shown in Figure 8 and Figures 10-10C, a method of providing a barrier to radiation is provided. The method includes inserting a non-sterile radiopaque insert 12 within a pouched sterile surgical drape 14. The drape 14 can be configured for various surgical procedures, and to accommodate different body shapes and sizes, with pouches located in different regions of the drape 14 and having different proportions relative to the size of the drape 14 depending on the anticipated location of radiation scatter and the area that must remain open for fluoroscopy. One drape 14 can be configured having a plurality of pouches, including pouches of different sizes and shapes, to accommodate various types of procedures, and the choice of insert 12 and corresponding pouches 14 to use are at the discretion of the surgeon. To further facilitate the reduction of the number of different drapes required in a procedure, pouches may configured to overlap other pouches to increase the variety of potential locations in which the inserts may be placed. Further flexibility of shield positioning may be afforded by placing fastening mechanisms, such as hook and loop straps, self adhesive strips, for example, that allow folding of portions of the drape into releasably fixed configurations, thereby allowing the assembly to take on a variety of configurations in use, and bringing various shield pieces into different positions, depending on the needs of the surgeon. In addition, to facilitate locating the assembly, the method includes providing a support mechanism or device and attaching or resting the mechanism upon the table-top or side-rails to provide partial or full support to the weight of the system so that the weight of the system it is not borne by the patient.

The inserts 12 may be quickly removed and replaced during procedures by the technologist using non-sterile hands, without disruption of the sterile field. This may facilitate lateral projections, extreme tube angles, etc.

Figure 8 depicts one embodiment of a pouched sterile surgical drape 14. The radiopaque shielding inserts 12 are inserted into integral pouches 37 of the drape, such as on the under-side of the drape 14, where it is non-sterile. Typically, sterile surgical drapes are applied over the patient such that the undersurface is non-sterile except in the area within and surrounding an access hole 40 in the drape 14, such as a femoral arteriotomy hole, for example. Since the majority of the underside of the drape 14 is not sterile, or is not required to be sterile, a non-sterile assistant may insert the non-sterile radiopaque inserts 12 into the selected pouches without contaminating the sterile field on an upper surface of the drape 14.

In Figure 9, a non-sterile insert 12 constructed in accordance with one aspect of the invention is shown disposed in a sterile outer drape 14. In contrast to other highly flexible insert embodiments of the invention, this insert 12 is rigid or semi-rigid or highly flexible such that it is plastically deformable to change between different configurations of rigid, semi-rigid, or highly flexible shape. It may be rigid along some axes, and flexible or semi-rigid along other axes, to permit the best combination of rigidity to facilitate insertion, and flexibility to conform to patient or surgical environment. A simple example of a semi-rigid insert 12 could be a leaded-fabric or vinyl flap, such as is used in lead aprons, surrounded by a nylon or other durable covering, and with a stiffener 38, such as a malleable metallic strip, contained within the Insert 12 to provide the insert 12 with a semi-rigid quality.

In Figures 10A-10B, one embodiment for a method of providing a barrier to radiation in a surgical procedure in accordance with the invention is shown, using the inserts 12 of Figure 9 in a pouched surgical sterile drape 14. The patient's skin is prepped for surgery (shown as dashed lines in the right groin region), and the drape 14 including an operative opening 40, in this case a circle, is placed on the patient P. The surgical drape 14 remains sterile on top (the sterile field) and is mostly non-sterile underneath once applied to the mostly non-sterile patient, who is only sterile in the prepared area within the region of the opening 40. The periphery of the drape opening 40 is fixed to the patient's skin, such as with an adhesive, to prevent movement of the drape 14 about the surgical region of the patient.

Figure 10B depicts the drape 14 of Figure 10A being lifted whereupon at least one insert 12 is placed within the desired pouch 37 on the under-side of the sterile surgical drape 14. The openings of the pouches 37 may then be closed to prevent inadvertent removal of the inserts 12, such as via a self adhesive, hook and loop fasteners, or some other closure mechanism.

As shown in Figure 10C, with the inserts 12 positioned within their respective pouches 37 on the under-side of the drape 14, the drape 14 may be dropped, and the inserts 12 may be plastically formed to shape as needed to allow the surgeon unfettered access to the surgical site on the patient P. In the embodiment illustrated, by way of example and without limitation, the inserts 12 are positioned and formed over the pelvis of the patient P, and hanging along the right side of the patient P to shield the surgeon, whom will also be at the patient's right side, from exposure to radiation scatter. The opening 40 in the sterile surgical drape 14 remains sterile to allow a sterile surgical procedure to be performed within the region of the opening 40.

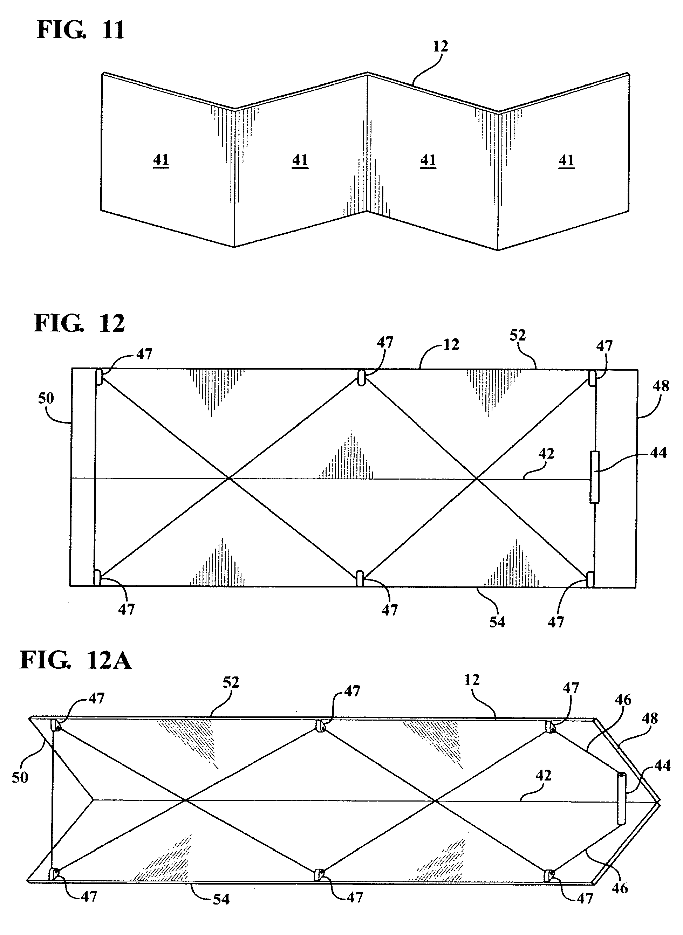

In Figure 11, an embodiment of a collapsible radiopaque shielding insert 12 constructed in accordance with another aspect of the invention is shown. The insert 12 can assume a lengthened, generally rectangular shape, or a plurality of individual rigid regions 41 can be foldable relative to another and upon themselves to assume a reduced, generally square shape. Of course, the individual regions 41 assume a wide variety of shapes and can also be entirely separate from one another whereupon they could be stacked for storage and placed adjacent one another, such as in overlapping relation, in use.

In Figure 12, an embodiment of a collapsible radiopaque shielding insert 12 constructed in accordance with another aspect of the invention is shown in a fully expanded state. The insert 12 has plurality of rigid sections foldable relative to another, shown as being foldable along a longitudinal central axis 42, which can be facilitated by pulling a handle 44 which applies tension to a filament 46 that is looped through the plurality of rigid sections such that the filament 46 is operable to bias the plurality of rigid sections into a folded configuration. The filament 46 is shown as extending through guides or eyelets 47 and between ends 48, 50 and sides 52, 54 of the insert 12. Upon applying tension to the filament 46, the opposite sides 52, 54 are biased toward one another, thereby causing the insert 12 to fold along the axis 42, such as shown in Figure 12A. As such, the width of the insert 12, when in its collapsed configuration, is half of its unfolded width. Then, when desired, the tension on the filament 46 can be relaxed, thereby allowing the insert 12 to resume it full width, which can be performed simply by unfolding the sides 52, 54 away from one another, similarly as a loose hinged door.

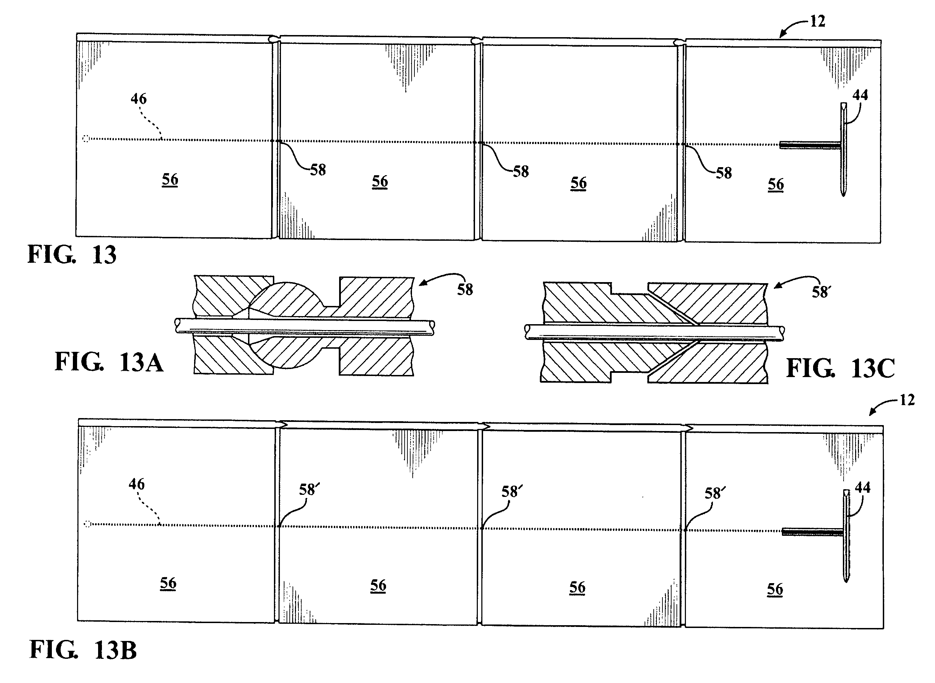

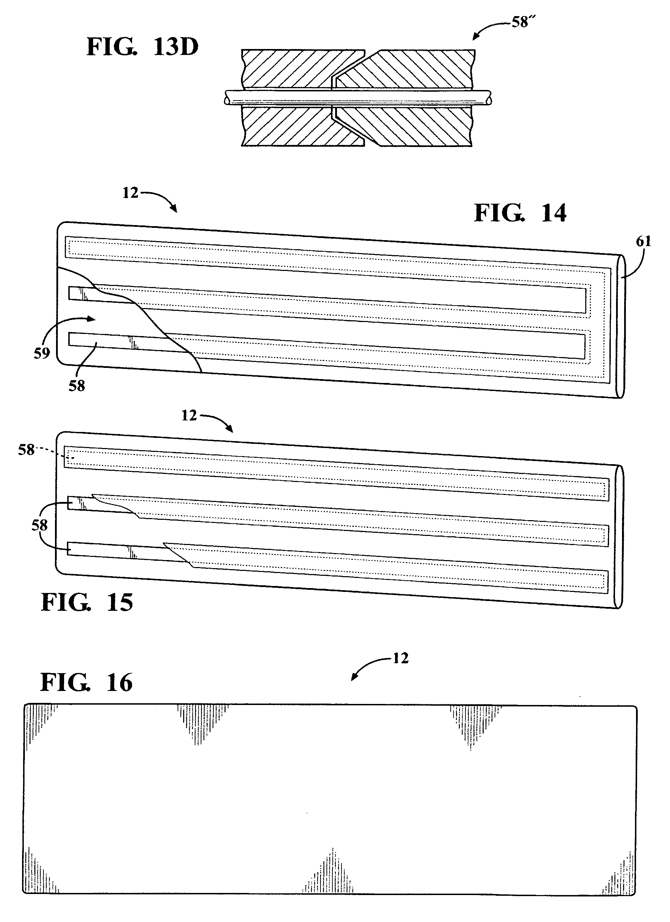

Figures 13 and 13B illustrate embodiments of a collapsible radiopaque shielding insert 12 constructed in accordance with another aspect of the invention shown in a fully expanded state. The inserts 12 have a plurality of hinged regions 56 to allow the insert 12 to be selectively manipulated to assume a locked, straight configuration, or a relaxed flexible configuration, wherein the individual insert regions 56 can be readily folded upon themselves. In the embodiments illustrated, the hinged regions 56 are interlinked with one another via a male and female hinges flexible joints that join adjacent ones of said plurality of rigid sections to one another. Each of the flexible joints or hinged regions 56 have a male member and a female member (shown having different configurations 58, 58' in Figures 13A and 13C, respectively, and an alternative configuration 58" in Figure 13D) that can be aligned or straightened to bring the insert 12 to a lengthened configuration, such as caused by tensioning a filament 46 running longitudinally along the insert 12 and through the hinges 58, 58'. When the filament 46 is tensioned, such as via a handle 44, the generally square sections 56 of the insert 14 components are drawn into a straight array that creates a flat or substantially flat rectangular sheet. When in the rigid lengthened configuration, the insert 12 is well suited for insertion into a pouch of a sterile drape 14, such as discussed above. Once the insert 12 is inserted into the pouch of the drape 14, the handle 44 is released, thereby releasing the applied tension on the filament 46, and thus, allowing the joints or hinges 58, 58' to flex freely. As such, the sterile insert and drape assembly may readily conform to the surface on which it rests, or hang to the side of the patient P or table as desired.

In Figure 14, an embodiment of a formable radiopaque shielding insert 12 constructed in accordance with another aspect of the invention is shown in a fully expanded state. The insert 12 includes a stiffener member 58, such as malleable rods that extend internally through the insert 12. The stiffener member 58 provides optimum semi-rigidity to the insert 12 and permit easy insertion of the insert 12 into a sterile drape, such as described above. In addition, the stiffener member 58 allows the insert and drape assembly, upon disposing the insert 12 into a drape 14, to be conformed to various body shaped and surgical table configurations. The stiffener member 58 may be permanently located inside the insert 12, or it may be removable, such as within a pocket 59 between opposite faces of the outer material forming the insert 12. As such, upon inserting the stiffened insert 12 into a pouch of a drape, the stiffener member 58 could be removed, such as via an opening 61, thereby allowing extreme flexibility of the insert and drape assembly 10.

In Figure 15, another embodiment of a formable radiopaque shielding insert 12 constructed in accordance with another aspect of the invention is shown in a fully expanded state. The insert 12 is similar to that describe and illustrated in Figure 14, however, and the insert includes a plurality of stiffener members 58 that are separate from one another. It should be recognized that the stiffener members 58 could be oriented along any desired direction and that they need not extend lengthwise along the insert 12. Accordingly, depending on the application, the insert 12 can be configured having the stiffener members 58 running in parallel or non-parallel relation with one another and can be configured to extend lengthwise, widthwise, or otherwise within the insert 12.

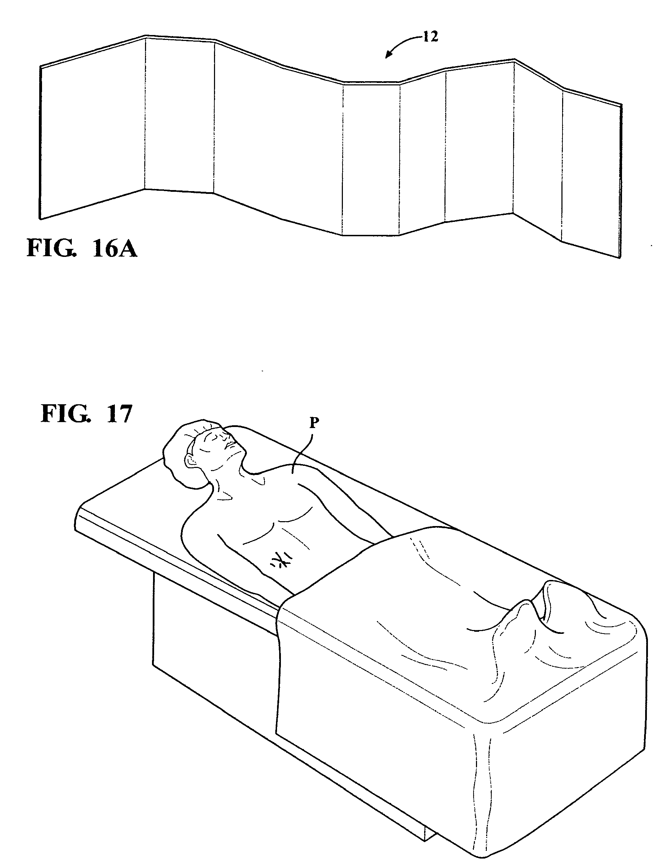

In Figures 16 and 16A, another embodiment of a formable radiopaque shielding insert 12 constructed in accordance with another aspect of the invention is shown in fully expanded and partially folded states, respectfully. The insert 12 includes an integrated mixture of a deformable substance and malleable substance into a plate-like form that is deformable, with semi-rigid physical properties. For example, the insert 12 could consist of a plate of lead inside a fabric skin, or a substrate impregnated with lead or other radiopaque compound could be used in lieu of the lead.

In accordance with another aspect of the invention, another method of constructing a radiation shield assembly 10 for providing a barrier to radiation in a surgical procedure is provided. The method can be referred to as a "layer method", in that various layers are placed over the patient to provide the desired shielding against scatter radiation to the surgical team. The method includes use of a first sterile inner drape 14 which can be formed having an opening 40 with a ring member 60 extending about the opening 40. The ring member 60 can have adhesive surfaces 62 both on a side facing the patient for adhesion to the patient and on a side facing upwardly away from the patient. In addition, the drape 14 can be provided having a fastener member, shown as a plurality of fastener members 64, located radially outwardly from the ring member 60, e.g. hook and loop member or self adhesive, on the side facing upwardly away from the patient. The method further includes use of a radiopaque layer 12, such as a flexible leaded sheet or blanket (or other radiopaque composition). Further yet, the method includes use of a second sterile outer drape 14'. The outer drape 14' could be provided as a standard angiographic drape made of paper or fabric and providing a barrier between sterile field on top, and potentially non-sterile objects underneath.

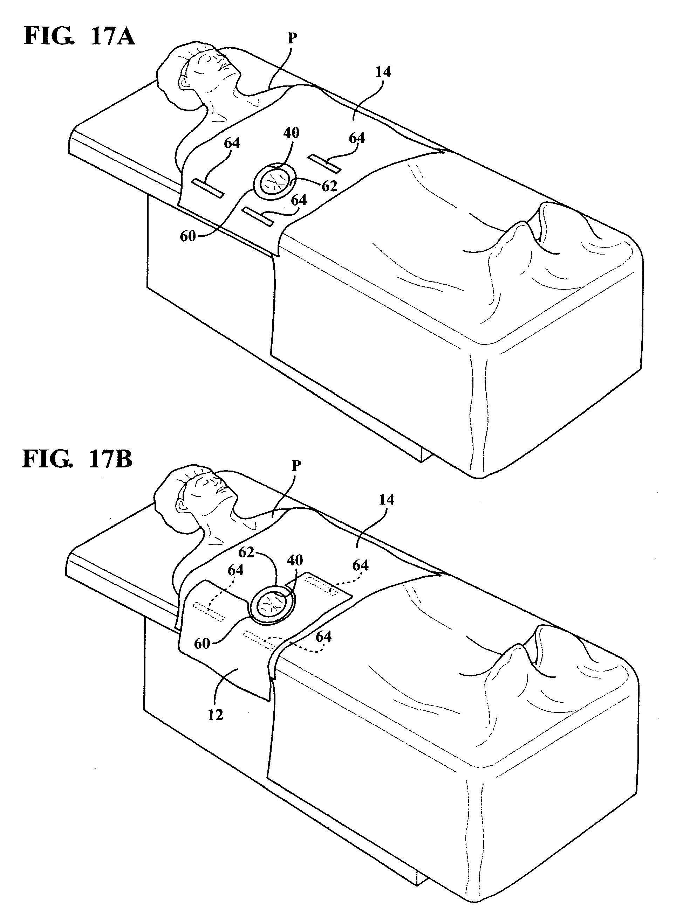

Figures 17A-17C disclose one embodiment utilizing the layer method. After scrubbing of the patient's operative area, shown by way of example and without limitation as being the right abdominal region, the inner drape 14 is laid over the patient P. Similar materials and methods may be used over areas of the body other than that depicted, such as commonly performed over the groin to access the femoral artery. The inner drape 14 has an opening 40 positioned over the abdominal region, which in this example has an adhesive surface, shown as a ring of adhesive tape 62 around it, both on an underside of the drape 14 to secure it to the patient's skin and on an upper side of the drape 14. It should be recognized that in some procedures, depending on the preference of the surgeon, the first drape 14 may not be used, thereby proceeding directly to the next step.

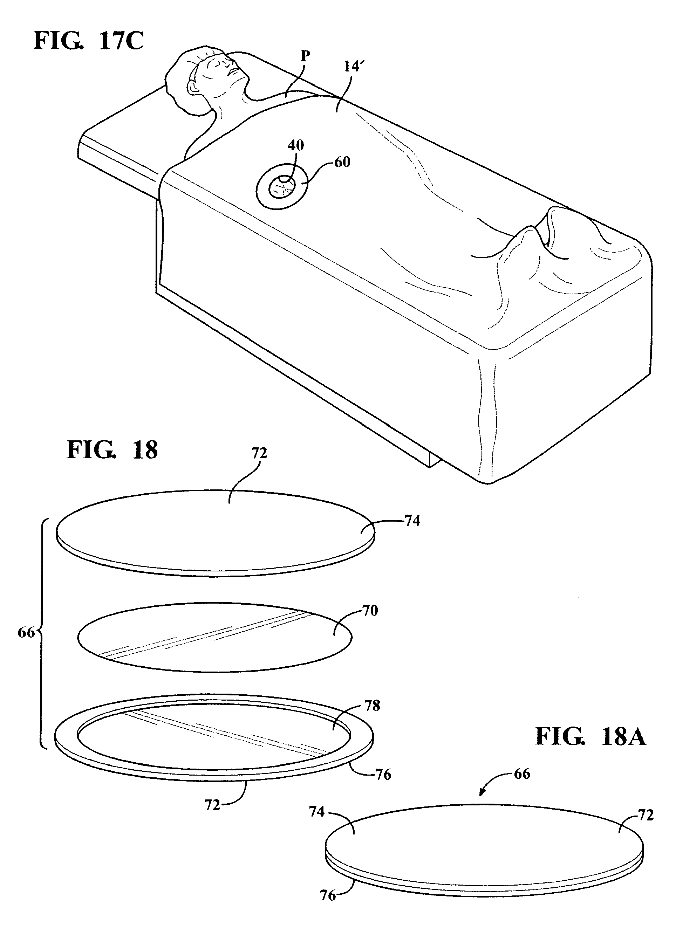

As depicted in Figure 17B, the flexible and/or formable radiopaque shield 12 is then laid over the inner drape 14 and stabilized or fixed to the inner drape 14 by fasteners 64 such as adhesive tape, or hook and loop fasteners (one of the hook or loop provided on the shield 12 with the other of the hook or loop provided on the drape 14), or some other conventional mechanism. Otherwise, the shield 12 may simply lay in position without fasteners, held in place by gravity and the friction of the mating surfaces, which may be enhanced with high friction materials. Conventional surgical drape clamps, known in the art, could also be used to fix the shield 12 to the drape 14. Then, as shown in Figure 17C, the sterile surgical drape 14' may be laid over the radiopaque shield 12, and the surgical procedure may commence through the opening 40 on the scrubbed skin. In the depicted embodiment, the opening 40 has an adhesive ring 60 around it, which adheres to the inner drape 14 beneath, thus adding to stabilization of the layered components so that relative slipping does not occur between the components which could compromise sterility, or alter radiowave-protective qualities. Alternatively, there may be an adhesive surface on only one of the two layers around the opening 40 (2 layers being the sterile drape 14' on top, and the underlying drape 14 in 17A). Since one drape has an adhesive surface, it will stick to the other drape not having an adhesive. Alternatively, there may be no adhesive, and it stays in place with surgical clamps, friction, or gravity.

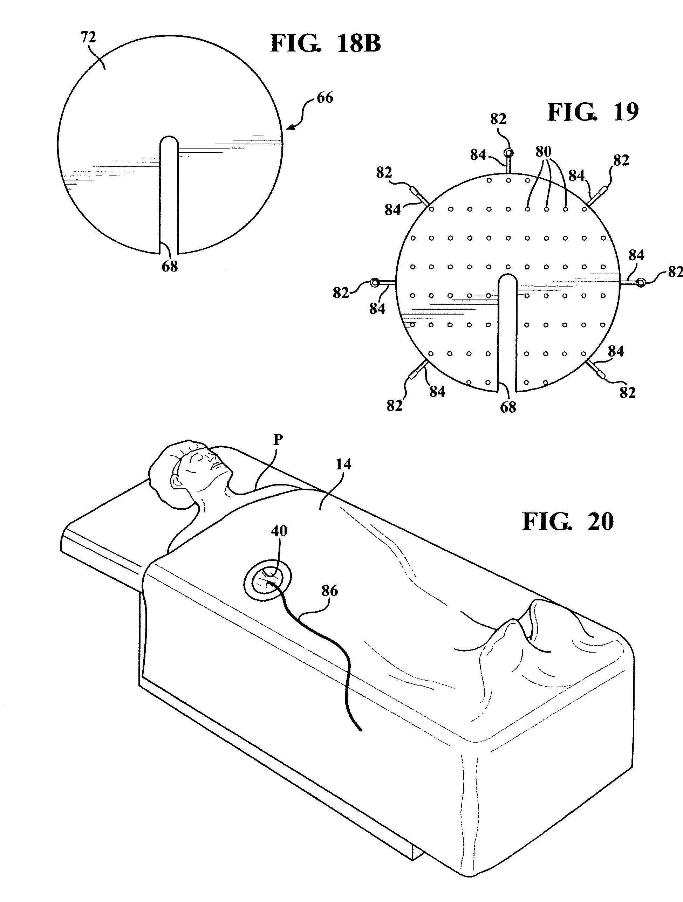

In accordance with another aspect of the invention, an apparatus and method is provided for provide shielding protection to a small area in the operative field corresponding to the opening in the drapes, where scatter radiation may emanate, and be particularly important with regard to protecting the surgeon's hands against exposure to radiation. As shown in Figures 18-18B, one embodiment includes an annular member, also referred to as hand guard 66 to obstruct and prevent radiation from passing through gaps or openings in surgical drapes through which a surgical procedure is being performed. The hand guard 66 has annular wall with an opening, also referred to as slit 68, extending radially outwardly from an approximate geometric center of the disc making the wall circumferentially discontinuous.

The hand guard 66 is designed to be capable of being positioned in the area or a drape surgical access opening 40 without hindering ability of the surgeon to retain full unfettered access to the surgical site. To facilitate blocking or shielding radiation, the hand guard 66 includes the opening, channel or slit 68 for passage of surgical tools. The hand guard 66 can be fabricated from sterilizable material, such that it can be reused, or it can be fabricated as a disposable single use device. To be re-sterilizable, it may benefit from being constructed of a metallic or plastic compound that tolerates high heat and pressure. Otherwise, it could have an outer shell of such sterilizable material to protect an inner core compound that could melt or decompose if exposed to the environment, or to extreme heat or pressure.

In Figure 18, one embodiment is shown that includes a radioprotective layer 70, in this embodiment comprising 0.5 mm Pb foil, which is encased in an outer stainless steel shell 72. The Pb foil could be injected into the shell 72 and sealed at the injection site, or it could be sandwiched between two discs of stainless steel 74, 76 that have a recessed pocket 78 between them to accommodate the layer of Pb or other radio-barrier composition. One or both discs 74, 76 include the recessed pocket 78 on one side, which can be machined, molded, laser cut, or otherwise formed, where a thin foil, powder, or other composition of lead or other x-ray barrier material is be disposed. The discs 74, 76 are placed together and sealed using welding, gluing, melting, or other form of bonding that creates an impervious barrier that is tolerant to high heat and pressures required for sterilization using common hospital techniques. Accordingly, the sealed joint provides a complete, impervious barrier or seal, and is not disrupted by the heat and pressure subjected within an autoclave (high pressure steam), or gas sterilization. The discs 74, 76 may also be manufactured from other materials such as ceramics, high strength plastics or other alloys suitable for the purpose described herein.

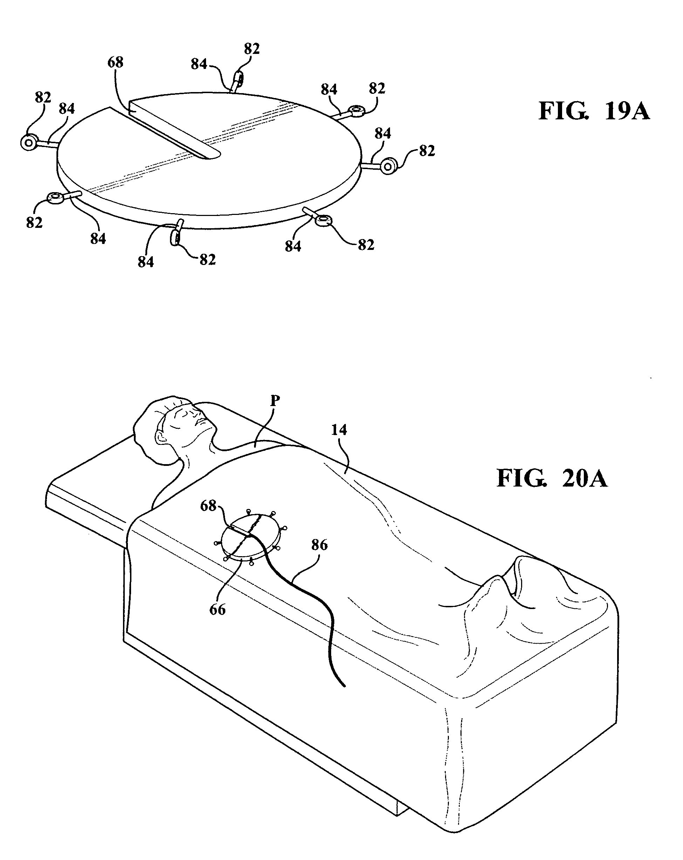

Figure 19 depicts one embodiment providing for the stabilization of a hand guard 66. The device 66 may tend to slide when placed on a slope, or if patient P moves. Stabilization can be provided in several ways in accordance with the invention. For example, a gripping friction material, represented as small feet 80, also referred to as spikes or pads, may be fixed on one planar side of the device 66, as shown. The feet 80 can be metallic, sharp, dull, or non-metallic, adhesive tape, glue pads, and hook and loop fasteners could be used as well. Since the material that this device rests on is usually paper fabric, or woven fabric drapes or sheets, sharp feet would tend to penetrate the fabric or deform it to some degree to provide stability. In addition to such mechanisms on the planar surface of the device, fastening mechanisms 82 along the outer periphery could be incorporated, shown as being attached to free ends of legs of malleable wire 84, for example. The legs 84 can be formed or plastically bent as desired to conform to the underlying surface. The fastening mechanisms 82 are configured as circular hoops that allow easy clamping of the hand guard 66 to the adjacent underlying material, such as with conventional surgical clamps (such as "towel clamps" which have sharp teeth, or "hemostats") that go through the hoop 82 and grip the fabric drape beneath. The circular hoops 82 can be provide to alternate in orientation with one another, thereby providing the surgeon with alternatives as to how to fix the device 66 in place. For example, alternating hoops 82 can be configured in a generally coplanar relation with the device 66, thereby having the openings through the hoops 82 facing in one direction, while the adjacent hoops 82 are oriented generally perpendicular to the intermediate hoop 82, such that the openings in the adjacent hoops 82 are oriented to face generally perpendicular to the intermediate hoop opening, as shown. Of course, if desired, given the legs 84 can be malleable, the surgeon could twist the hoops 82, as desired, to allow the device 66 to be readily fixed in place via the chosen fastening mechanism applied to and/or through the hoops 82.

Figure 20 illustrates a patient P that has been prepared and draped with sterile surgical drape 14. There may be inserts 12 beneath the drape, as discussed above. A hand guard 66, such as shown in Figure 20A, which has been sterilized, is placed over an opening 40 where an instrument, such as a catheter and sheath 86 have already been inserted into the patient P. As shown, the hand guard 66 has a slit 68 that accommodates the sheath and catheter 86.

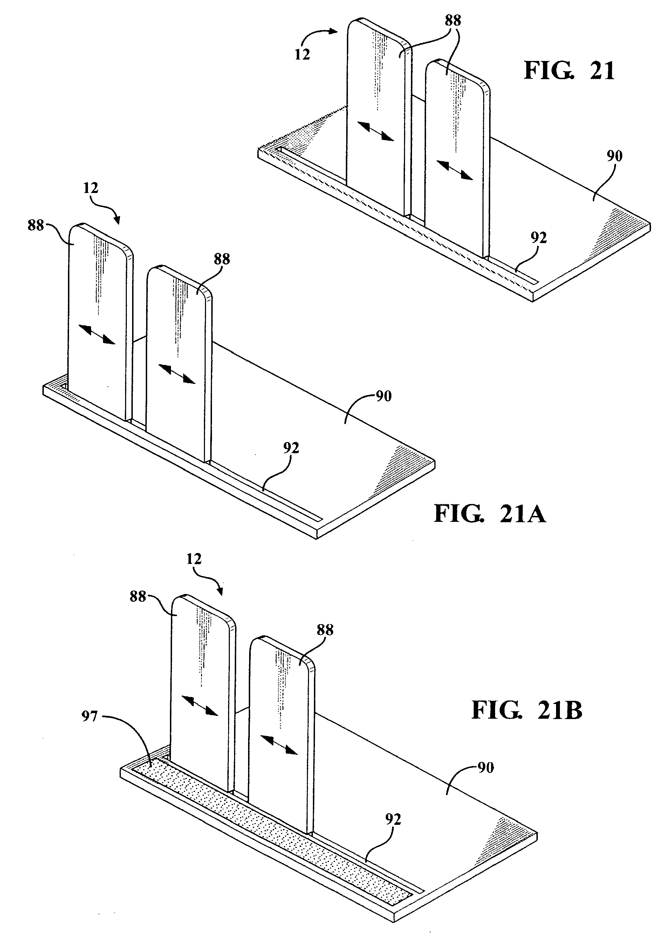

In Figures 21 and 21A, another embodiment of a radiopaque shielding device 12 constructed in accordance with another aspect of the invention is shown. The device 12 is suitable where blankets or hand guards are difficult to position, such as due to gravity presenting complications, and also where fastening mechanisms for a drape might result in pulling or distortion of its desired shape or position. Some surgical procedures require access to the side of a patient. This embodiment addresses this by being supported along a side of the operating table, and having a vertical or substantially vertical orientation. The device 12 includes a plurality of rigid shielding elements, also referred to as shields 88, constructed of radiopaque material. The shields 88 are shown as being configured to slide relative to one another laterally along a base 90, such as in recessed tracks 92, wherein the tracks 92 are configured to stabilize the shields 88 in their upright, generally vertical orientation.

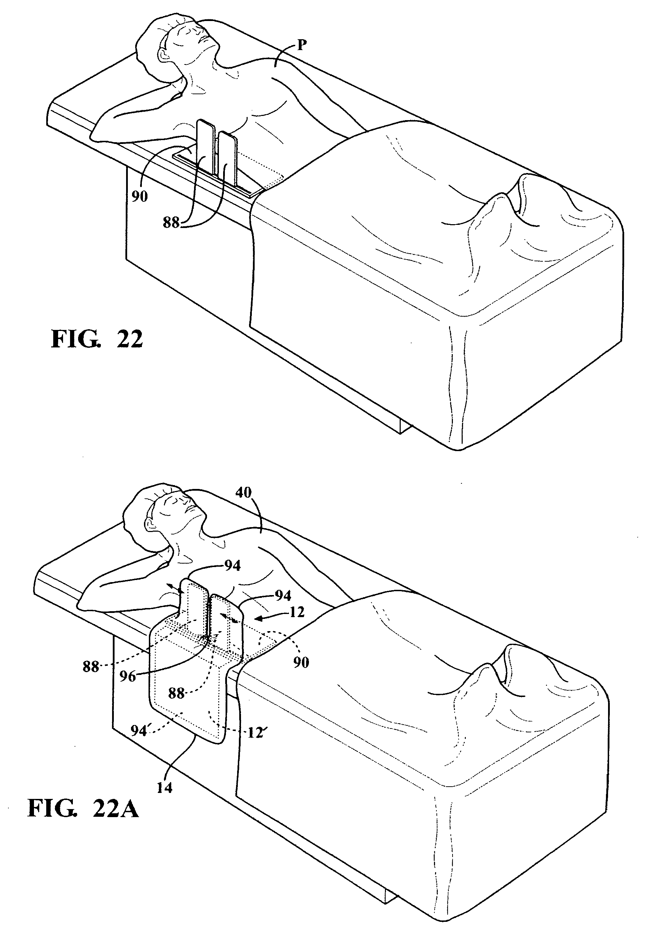

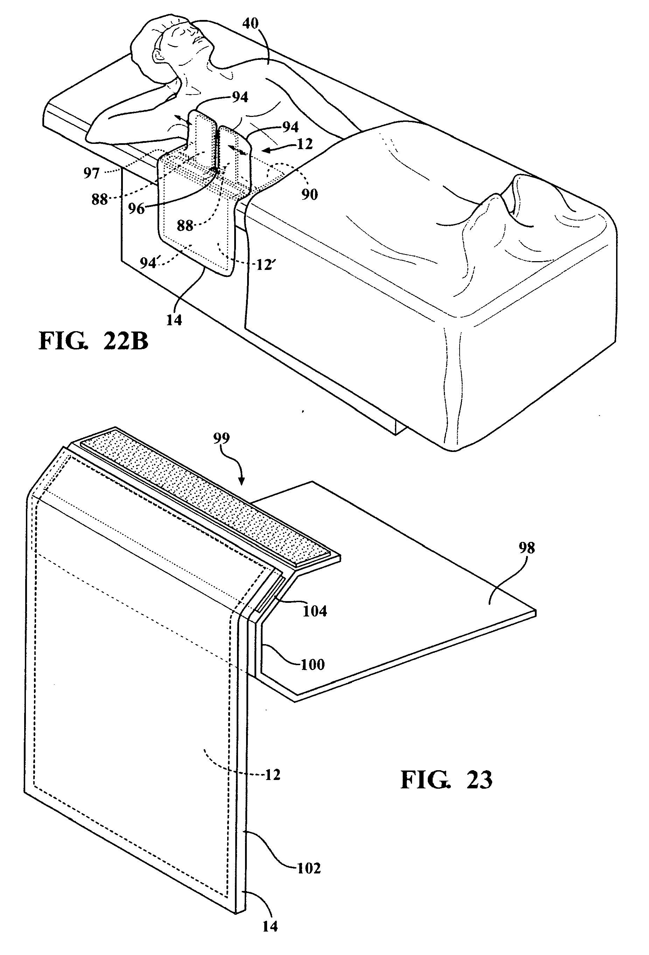

Figures 22A and 22B show the sliding elements or shields 88 and base 90 configured at right angles to one another, thereby permitting the base 90 to be slid or otherwise positioned beneath a patient P. As such, the upstanding shields 88 are stabilized by the weight of the patient P fixing the base 90 in its positioned location. Further stabilization could be provided by conventional fastening mechanisms of the base 90 to the table, if desired, and are contemplated to be within the scope of the invention. With the base 90 fixed beneath the patient P, the shields 88 can be slid to their desired position along the tracks 92. In Figure 22A, the shielding device 12 is shown having a sterile drape 14 disposed thereover, with the drape having separate pockets 94 configured to slide over the laterally spaced shields 88. Each pocket 94 is shaped similarly to the respective shield 88 that is received therein. The pockets 94 are spaced from one another via gathered material 96 allows the pockets 94 to move freely with the shields 88 as they are slid along the tracks 92. Upon the shields 88 and corresponding pockets 94 being translated along the tracks 92, the desired size open space between the shields 88 is provided to permit access to the surgical site on the patient P. The sterile drape 14 can be further provided with a pocket 94' configured to depend from the base 90, such that the pocket 94' can hang downwardly from a surgical table, for example. The pocket 94' is sized for receipt of a radiopaque insert 12', such as those described above. In other embodiments, such as shown in Figures 21B and 22B, the radiopaque sheet 12' is detachably attached to the base 90, such as via a fastener 97, which can include, by way of example, one portion of a hook and loop type fastener for attachment of the mating portion of the hook and loop fastener affixed to the radiopaque member 12'. Of course, other types of fasteners, such as those discussed above, could be used to releasably fix the radiopaque member 12' to the base 90. Then, the radiopaque sheet 12' can be covered by sterile drape 14, wherein the sterile drape 14 can be configured as a one-piece drape, thus being configured for receipt over the upstanding shields 88 and to cover the sheet 12', or as a separate drape configured to cover just the radiopaque sheet 12', wherein another drape can be used to cover the upstanding shields 88. Further, it should be recognized that the radiopaque sheet 12' could be permanently fixed to the base 90, if desired. The shielding device 12 may be used in conjunction with other sterile surgical drapes (not shown), such as those described above and illustrated. It should be recognized that the shielding device 12 can first be draped with a sterile drape 14, and then the draped shield assembly 10 may be pushed or otherwise positioned under the patient P, thus maintaining sterility throughout the surgical procedure area. Further, it should be recognized that instead of the sterile drape 14 being provided as a monolithic piece of material, it could be provided as separate, modular components. For example, a single sterile drape could be provided to cover the upstanding shields 88, and a separate sterile drape 14' could be provided, with a radiopaque shield 12' therein, for attachment via any suitable mechanism, such as those discussed above with regard to modular components, to the base 90 of the shield 12, or any other suitable location. Further yet, separate, individual pouched drapes could be used for each upstanding shield 88.

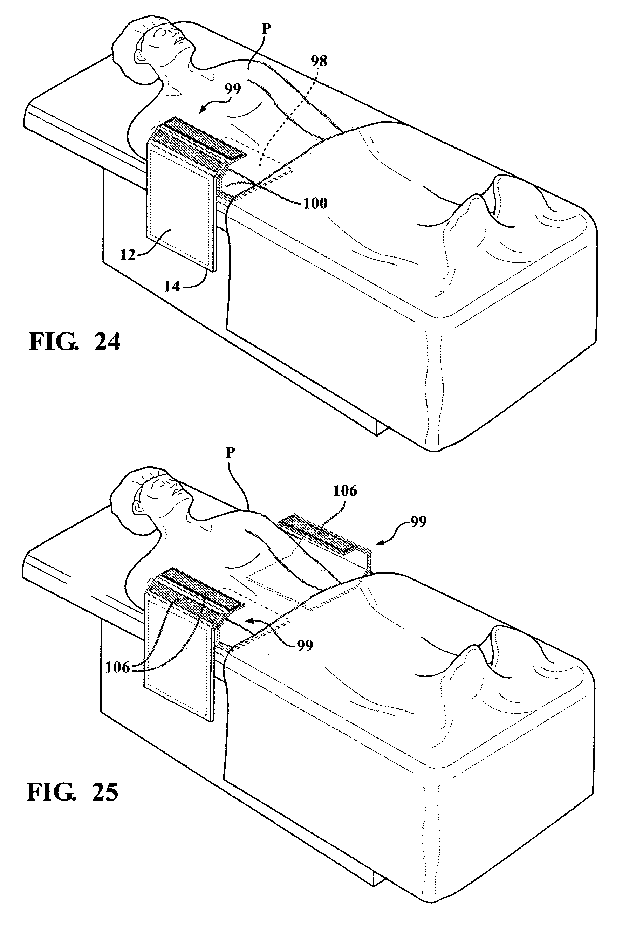

In Figure 23, yet another embodiment for deploying a radiopaque shield 12 is constructed in accordance with the invention is shown. A generally rigid support member, referred to as an arm board 99, has a curved shape and has a generally flat base portion 98 that slides under a patient P (Figure 24) for stability and an upstanding sidewall, shown as a curved portion 100, shown a being generally channel or C-shaped. The curved portion 100 is configured to wrap about a side of the patient P wherein it may serve to maintain an arm of the patient in place and prevent the arm from hanging over the side of the table. Of course, any suitable mechanism, other that sliding the base 98 under the patient, for attaching the support member 99 in its desired location is contemplated to be within the scope of the invention, e.g. clamps, structures under the surgical table, or otherwise. The arm board 99 may contain radiopaque materials throughout its structure, for example either as Pb-acrylic, or a lead foil impregnated within it, or lead-vinyl strips layered with a rigid or semi-rigid substance. The arm board 99 may not necessarily be transparent to visible light, although some materials such as Pb-acrylic, could be use and provided as being transparent, thereby facilitating unobstructed viewing the surgical site. It should be recognized that the arm board 99 may not contain radiopaque materials throughout, such that the radiopaque materials could be located, as needed, in certain regions of the arm board 99 to permit suitable protection against radiation while not blocking direct beam for certain functions or tube angles during the surgical procedure. The arm board 99 may be constructed to support the flexible radiopaque sheet, also referred to as shield curtain 102, which can be comprised of an inner radiopaque insert 12 and an outer sterile drape 14, wherein the curtain 102 is configured to hang freely from the arm board 99 and extend to a free end for added protection against radiation scatter. The curtain 102, by way of example, can be secured to the arm board 99 via any suitable fastener 104, such as an adhesive, hook and loop fastener, or otherwise. Further, the arm board 99 can be provided having a fastener, such as an adhesive, hook and loop fastener, or otherwise, for attachment of secondary devices thereto, such as a sterile drape 14, for example. Otherwise, the sterile drape 14 can be provided having a pocket for receipt of the radiopaque shield 12 therein.

In Figure 24, the arm board 99 is shown slid under the patient P for stabilization and positioning. The patient's arm is shown positioned along the patient's side and contained within the upstanding curved portion 100 of the arm board 99, thus, preventing the arm from falling over the edge of the surgical table. The arm board 99 could be placed under a sterile surgical drape 14, and therefore would not need to be sterile itself. Or, if applied over a sterile surgical drape, it could be wrapped in its own sterile drape. If applied under a drape, it could be placed or removed by non-sterile personnel by lifting the drape. If applied over a drape, a sterile operator could manipulate it directly.

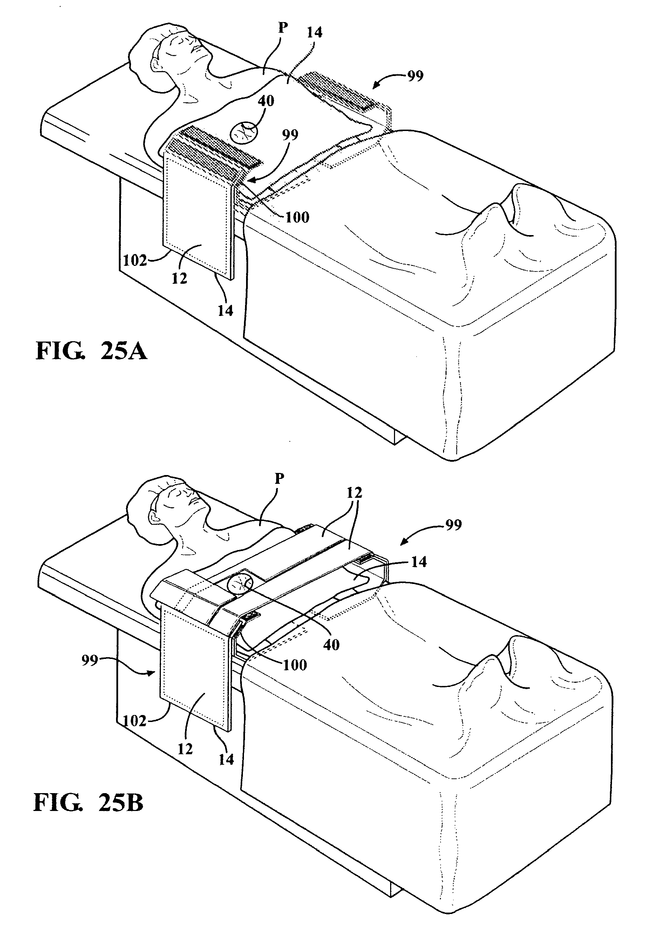

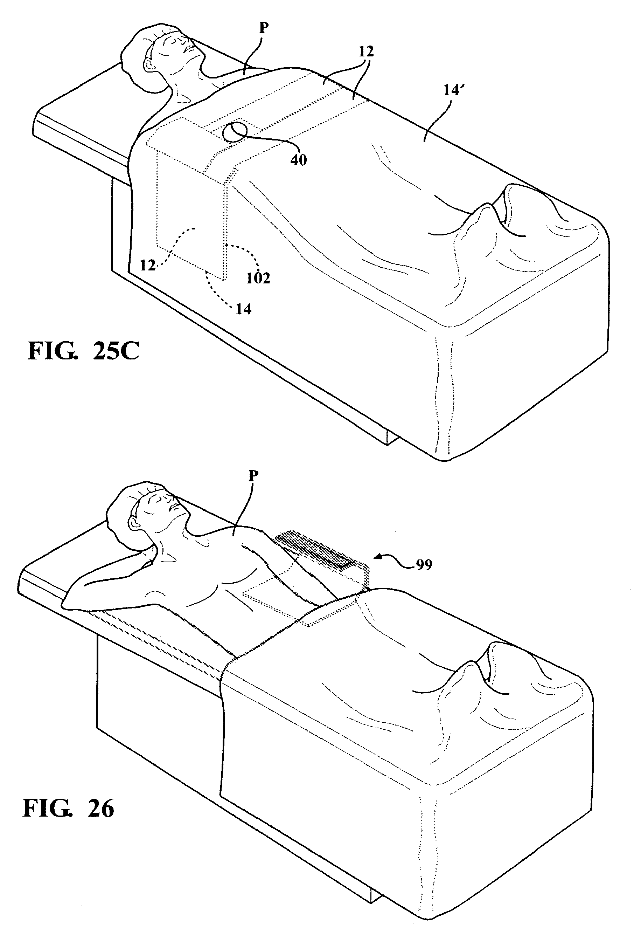

Figures 25-25C illustrate another embodiment including a pair of support members 99 discussed above with regard to Figure 24, wherein a layer method of applying a radiopaque shield 12 to the arm boards or support members 99 is utilized. The arm boards 99 are placed in position beneath the patient's P opposite sides, as shown. In Figure 25A, as shown, a first inner sterile or clean sheet or drape 14 is placed on the scrubbed patient P, containing an opening 40 for patient access, in this case over the lower abdomen region. It should be recognized that in some procedures, depending on the preference of the surgeon, the first drape 14 may not be used, thereby proceeding directly to the next step.

As shown in Figure 25B, a radiopaque sheet or shield 12 is layered over the inner drape 14, and over upstanding ends of the curved portion 100 of the arm board 99. The fasteners 106, such as hook and loop fasteners, stabilize the overlying radiopaque shield 12 to the ends of the upstanding curved portions 100. Part of the weight of the radiopaque shield 12 may also be resting on the patient P. As shown in Figure 25C, a second sterile surgical drape 14' may then be applied as shown previously with layer method. Note is made that the radiopaque shield 12 may be laid, or removed, after the application of the sterile drape 14' as well, by a non-sterile assistant who may reach under the drape 14' and reposition objects as needed. In this depiction, the arm board 99 is not radio-protective, and may thus be left in place regardless of tube angle without interference with direct beam.

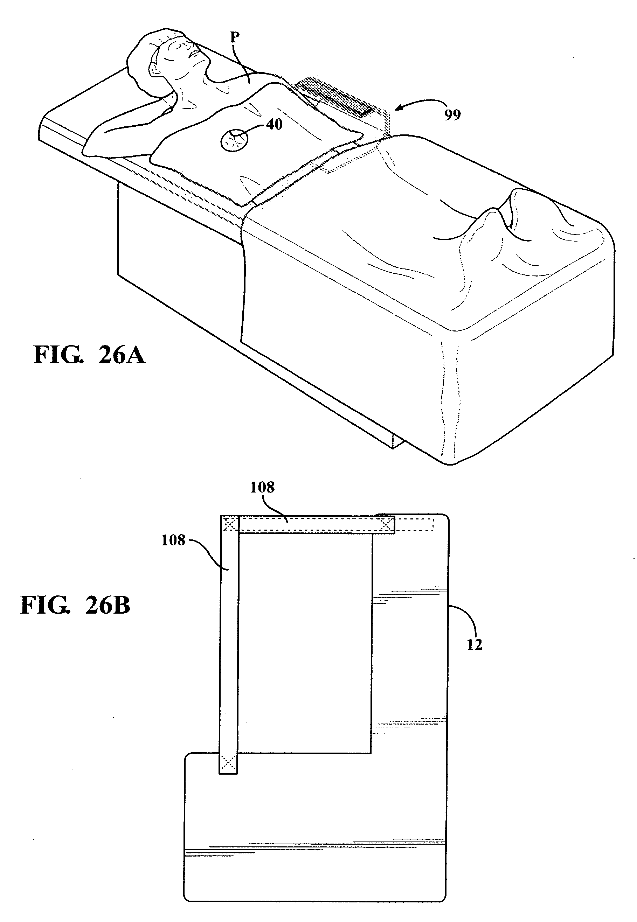

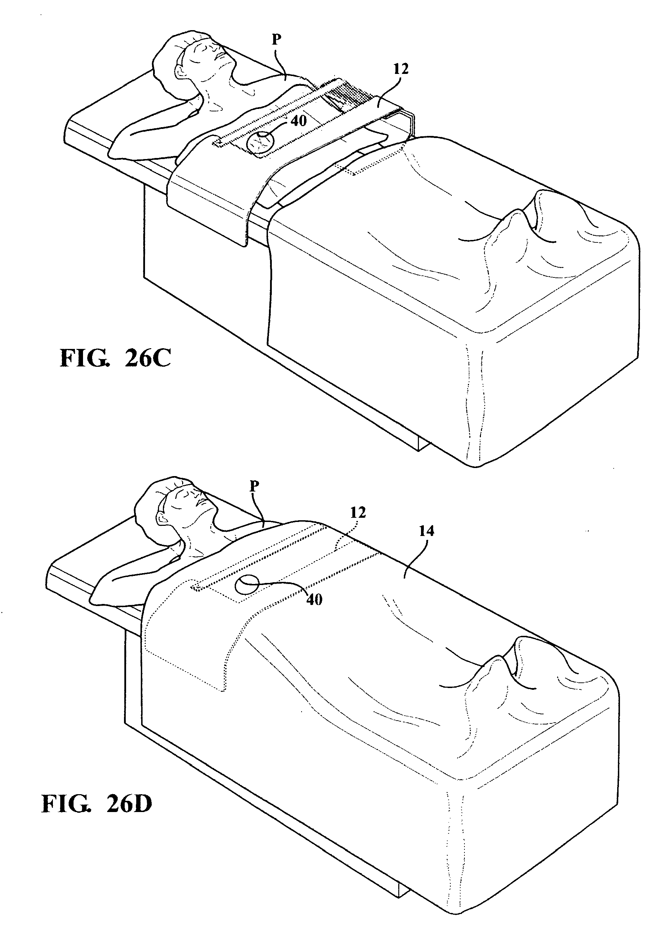

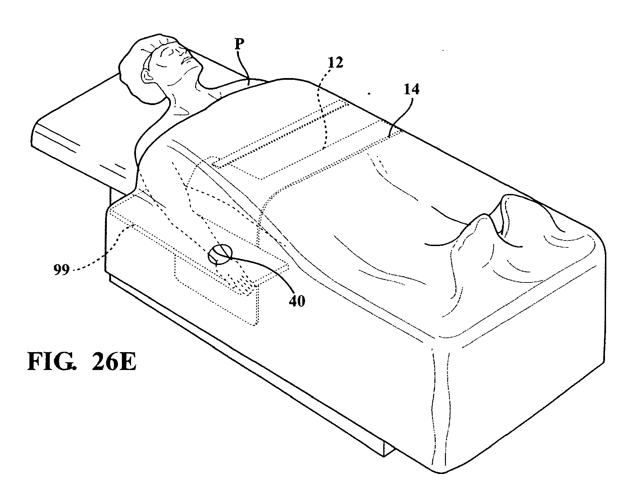

Figures 26-26D illustrate a flexible radiopaque shield 12 laid over the arm board 99 and fastened to it. The shield 12 may hang over the right side (side nearest in view), but is stabilized against falling or otherwise shifting by the arm board 99 positioned along the left side (side further from view). In this configuration of the shield 12, sagging and other unwanted movement of the shield 12 is prevented by the addition of a radio-lucent strap 108 extending from one side to the other, as shown.

As shown in Figure 26C, with the radiopaque shield 12 being draped on patient's right side to the level of the table-top, the radio-lucent strap 108 does not interfere, as there is no lead or other radiopaque material in the path of the imaging beam. The drop-down is done quickly and easily by the technologist from the opposite (left) side of the table (furthest side from view) with non-sterile hands. The technologist may reach under the sterile drape 14, and pull the radiopaque shield 12 and its radio-lucent strap 108 off of the hook and loop fastener on the arm board 99, feed it forward to drop the radiopaque shield 12 on the right, and then lay it back against the hook and loop fastener to secure the new position. For this purpose, the unilateral arm board 99 anchor system may have advantage over the aforementioned bilateral arm board attachment, because the technician can more easily perform the lowering of the shield 12 from the other side without having to re-do the attachments on the ipsilateral side as well, since it may simply slide down.

One embodiment of the shape of radiopaque shield 12 is shown in more detail in Figure 26B. The radiopaque shield 12 may have hook and loop fasteners or some other fastener or adhesive in numerous locations to adhere to supportive arm board 99. Many other shapes or designs of the radiopaque shield 12 are possible, all of which may provide the function of laying in a desired configuration on the patient.

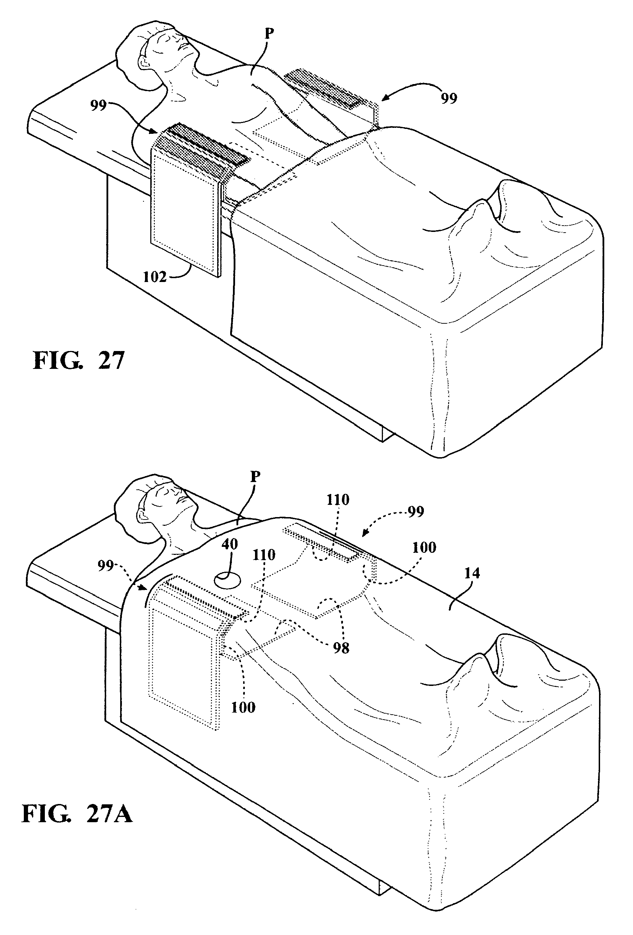

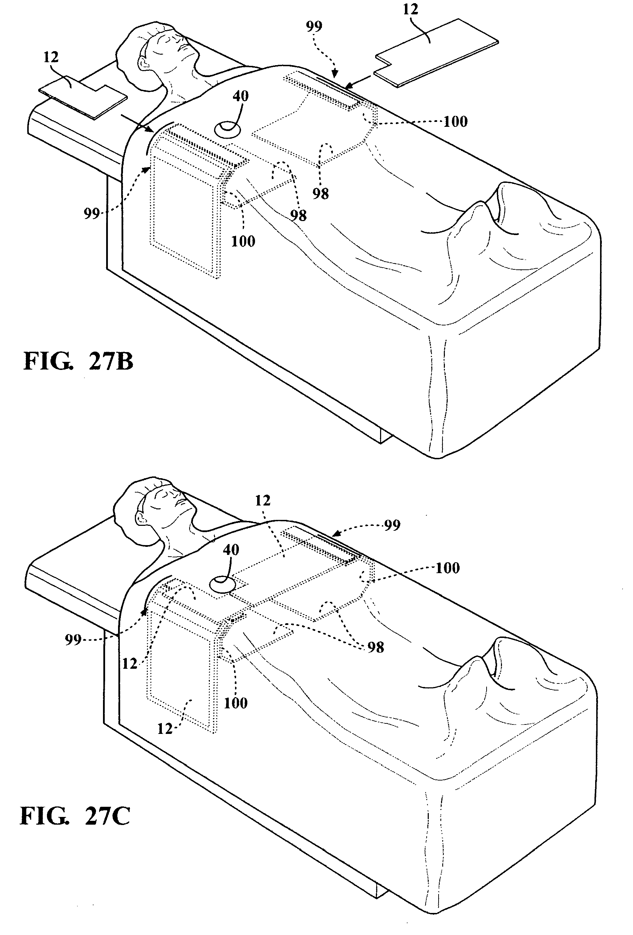

Figures 27-27C depict an embodiment of the modified arm boards 99 to provide stabilization for the radiopaque inserts 12 using an insert method, as discussed above. In Figure 27A, the base 98 of each non-radiopaque arm board 99 is placed beneath the patient P and the curved portions 100 are positioned to face one another such that they wrap toward one another. A radiopaque sheet or curtain shield 102 is fixed to a portion of one of the arm boards 99, shown as the right arm board 99 (nearest in view). In accordance with another embodiment, the hanging radiopaque sheet 102 may not be present, and instead this area would be shielded using suitably shaped inserts 12, such as those described above. A free end of the curtain shield 102 hangs freely from the right arm board 99 under the force of gravity. Then, as shown in Figure 27A, a pouched sterile surgical drape 14 (described previously as having pouches, also referred to as pockets, sized and configured to receive various shapes of radiopaque inserts) is laid over the scrubbed and prepared patient P. The drape 14 illustrated includes fasteners 110, such as one portion of a hook and loop fasteners for attachment to the other of the hook and loop fastener on the arm board 99, or other types of fasteners, such as an adhesive, on the underside of the drape 14 that secure the drape 14 against relative movement with the arm boards 99.

Upon fixing the drape 14 to the arm boards 99, as shown in Figure 27B, a portion of the drape 14 is lifted and the radiopaque insert(s) 12 are inserted into their respective pouches of the drape 14. The inserts 12 provide a barrier to radiation scatter, while the drape 14 is stabilized against movement, and thus, inhibited from sagging or moving under the weight of the inserts 12 due to stabilization provided by being fixed to the upper ends of the arm boards 99. In Figure 27C, the semi-rigid, pliable inserts 12 are plastically deformed, and thus, shaped to conform as desired to facilitate unobstructed access to the patient during the surgical procedure, shown here as extending across the lower torso of the patient and also hanging down within a pocket of the drape 14 on the right side of the patient and surgical table. In the event that the inserts 12 interfere with the procedure due to change in image receptor angle or other change, the insert(s) 12 may be easily removed by non-sterile personnel by sliding the insert(s) out of the pocket(s) of the drape 14.

In yet another embodiment, as shown in Figure 26E, which may be particularly suitable when the surgeon is accessing an artery in the arm or wrist of the patient P, where the sterile access to the patient's skin would be located, the patient's right arm, for example, could be placed down along the side of the patient, and could be located outside (above or external to; also could be said to be lateral, or to extended to the patient's right) of the radiopaque sheet 12. The radiopaque sheet 12 can be configured as desired, to allow imaging of the desired area of the patient, show as being generally the same as discussed with regard to Figure 26B, because fluoroscopy would be mostly performed in the chest region for a chest related procedure, e.g. heart procedure, and produce scatter even though the access point is outside of the radiopaque sheet 12. In this manner, the right arm would be held in position by an arm board or other suitable support device 99 that holds the arm above and lateral to the radiopaque sheet 12. The sterile drape 14 may be placed over the arm and body in the same manner as already depicted and discussed to provide its intended functions, as discussed above. In other embodiments, where the arm is the access site and also the subject of the surgical intervention, such as when treating a dialysis graft, the materials and principle components of the invention discussed and shown herein may be used in alternate designs that integrate or utilize the operating table arm support structures similarly to those depicted and described for the torso. It should be recognized that depending on the type of surgical procedure being performed, it may be advantageous to use the principles of this invention to provide separate mechanisms/configurations of stabilization of radiopaque device and arm.

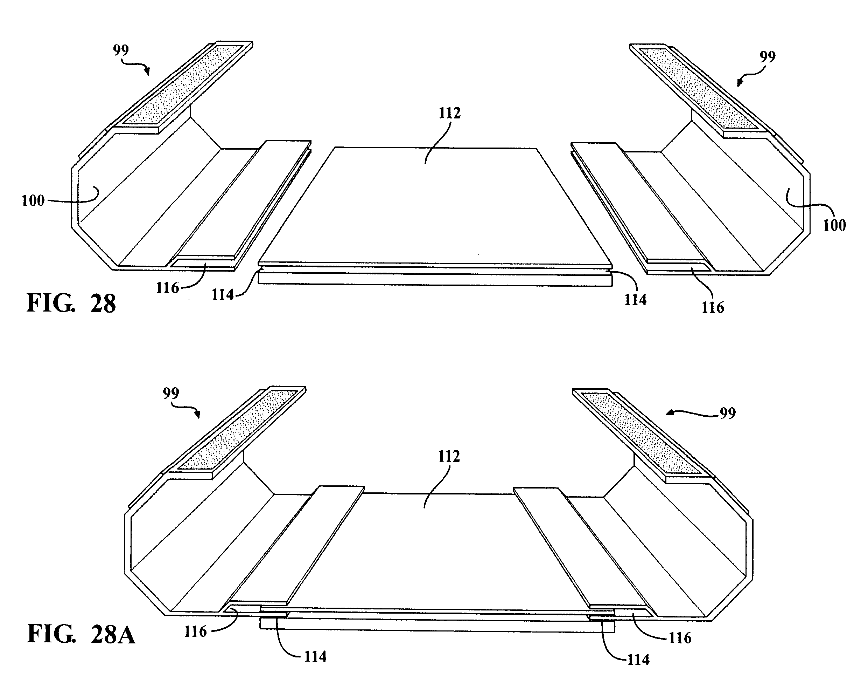

In Figures 28 and 28A, another embodiment of the rigid support members 99 is shown that are similar to the arm boards 99 discussed above, however, they do not rely entirely on the patient's weight for stabilization. The arm boards 99 include a central base 112 that is a separate piece of material from the arm boards 99. The base 112 is configured having an attachment mechanism or feature 114 to allow an attachment mechanism or feature 116 on the separate arm boards 99 to be releasably fixed thereto. The attachment feature 114 on the arm boards 99 is illustrated, by way of example and without limitation, as being one of a tongue or groove, shown as a groove 114, sized for receipt of the other of the tongue and groove 116 extending along an edge of the arm boards 99. As such, the base 112 can be positioned as desired on a surgical table and then one or both of the arm boards 99 can be fixed to the base 112 prior to the patient being placed on the table (Figure 28A). Then, the radiopaque shield(s) 12 can be fixed to the arm board(s) 99, such as via fastening members adjacent free ends of the upstanding sidewalls 100; the patient laid over the base 112, and then the sterile drape 14 disposed and fixed to the arm board(s), as described above. Of course, the drape 14, if pouched, can be first attached to the arm board(s) 99 and then the radiopaque inserts 12 can be disposed in their respective pocket, if desired, either before or after the patient is resting on the base 112.

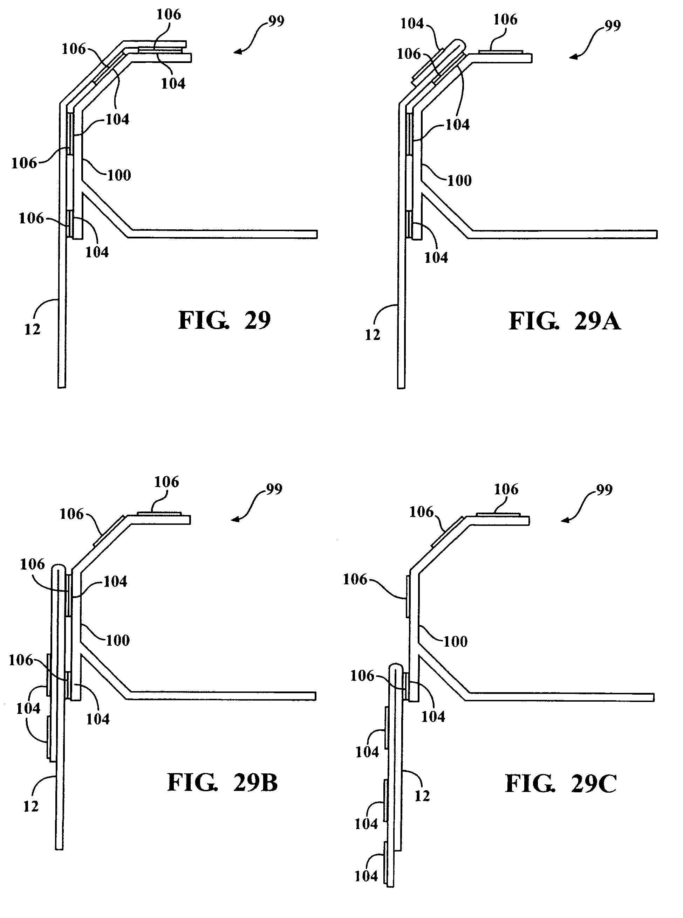

In Figures 29-29C, another embodiment of the arm boards 99 discussed above is illustrated, wherein the additional features discussed here can be applied to any of the arm board embodiments discussed above. The notable difference here is with regard to fastener members 104 on the radiopaque shield 12 and fastener members 106 on the arm board 99. Each the radiopaque insert 12 and arm board 99 have a plurality of corresponding fastener members 104, 106 to allow the insert 12 to completely cover the curved portion 100 of the arm board 99, or to be selectively unfolded away from a plurality of sections of the curved portion 100 adjacent a free end of the upstanding sidewall, while remaining fixed to a lowermost section of the arm board 99 sidewall, or to be detached completely from the arm board 99 (not shown). As such, with the arm board 99 being constructed from non-radiopaque material, the curved portion 100 can be substantially unshielded by the radiopaque insert 12, as desired, to allow an image of the patient to be obtained through the uncovered portion of the curved portion 100. Of course, only the necessary portion of the curved portion 100 need be uncovered, while leaving the remain sectors of the curved portion 100 to remain covered for maximum protection against radiation scatter.

Many modifications and variations of the present invention are possible in light of the above teachings. It is, therefore, to be understood that the invention may be practiced otherwise than as specifically described, wherein the various embodiments discussed may be used in combination with one another and reconfigured as desired for the intended surgical procedure. Further, it is to be understood that though possibly not expressly described in the written detailed description, the drawings in and of themselves constitute written description, and as such, terminology to describe what is shown in the drawings may be added without constituting new matter. It is to be further understood that the scope of the invention is defined by any ultimately allowed claims.

## Claims

1. A radiation shield assembly (10), for protecting an operator from scatter radiation, comprising:
a flexible drape (14) having a sterile outer surface and at least one through opening; the flexible drape (14) having at least one pocket (37; 94; 94') and
at least one radiopaque member (12) being disposable in the at least one pocket (37) and arrangeable about said at least one through opening (30; 40);
wherein the at least one pocket (37 94; 94') is arranged to have an opening (16), which allows insertion of the radiopaque member (12), and a fastener (18), wherein the opening (16) is closable by the fastener (18) in an air/fluid tight manner.

2. A radiation shield assembly (10) according to claim 1 wherein the opening (16) is arranged along an edge of the drape.

3. The radiation shield assembly (10) of claim 1 wherein said flexible drape (14) has a plurality of pockets (37 94; 94') and a plurality of radiopaque members (12) disposed in said pockets (37 94; 94') and preferably said radiopaque members (12) substantially surround said at least one through opening (30; 40).

4. The radiation shield assembly (10) of claim 1 wherein
(i) said at least one radiopaque member (12) includes a plurality of rigid sections (56) foldable relative to another or
(ii) said at least one radiopaque member (12) is plastically deformable.

5. The radiation shield assembly (10) of claim 4 further comprising an adhesive surface surrounding said at least one through opening (30; 40).

6. The radiation shield assembly (10) of claim 4 option (i) further including a flexible joints (56) joining adjacent ones of said plurality of rigid sections to one another, each of said flexible joints (56) having a male member (58) and a female member (58').

7. The radiation shield assembly (10) of claim 4 option (i) wherein said radiopaque member includes a filament (46) looped through said plurality of rigid sections (56), said filament (46) being operable to bias said plurality of rigid sections (56) into a folded configuration and preferably further including a handle (44) attached to said filament (46).

8. The radiation shield assembly (10) of claim 1 wherein said at least one radiopaque member (12) has an annular wall with an opening (38) making said wall circumferentially discontinuous.

9. The radiation shield assembly (10) of claim 8 wherein said drape (14) is configured being similarly shaped as said annular wall of said at least one radiopaque member (10) and further including a strip of material (32) bridging said opening of said circumferentially discontinuous wall.

10. The radiation shield assembly (10) of claim 9 wherein said drape (14) has opposite faces and further including gripping friction material attached to at least one of said faces.

11. The radiation shield assembly (10) of claim 1 wherein the radiopaque member (12) is operably connected to a rigid support member, the rigid support member (98; 99) having a substantially flat base portion (90; 98) and at least one upstanding sidewall (88; 94; 100) and optionally the side wall comprises a pair of upstanding side walls (88) each of the sidewalls being detachable from the base (90; 98) or the flat base portion (90; 98) is arranged to slide under the patient in use and the upstanding sidewall comprises a curved portion (100).

12. The radiation shield assembly (10) of claim 11 wherein the radiopaque member (94) hangs freely from the side wall (90: 98) and may be releasably fixed to the side wall (90: 98) by a plurality of discrete fasteners (18).

13. A method of providing a sterile barrier to radiation in a surgical procedure, comprising:
laying a flexible drape (14) having a pocket (37 94; 94') arranged to have an opening (16), which allows insertion of at least one radiopaque member (12), and a fastener (18), wherein the opening (16) is closable by the fastener (18) in an air/fluid tight manner, the drape having a sterile outer surface and at least one through opening (30; 40) over a patient; and
disposing the at least one radiopaque member (12) in the pocket (37 94; 94') about the at least one through opening (30; 40).

14. The method of claim 13 further including providing the drape (14) with a plurality of pockets (37 94; 94') disposing a plurality of radiopaque members (12) in the pockets (37 94; 94').

15. The method of claim 13 further including either
(1) plastically deforming the radiopaque member (12) after disposing the radiopaque member in the pocket (37 94; 94') or
(2) folding the radiopaque member (12) before disposing the radiopaque member (12) in the pocket (37 94; 94') and unfolding the radiopaque member (12) after disposing the radiopaque member in the pocket (37 94; 94').

## Patentansprüche

1. Eine Strahlungsschutzanordnung (10) für den Schutz eines Bedieners vor Streustrahlung, die Folgendes aufweist:
eine flexible Schürze (14) mit einer sterilen Außenfläche und mindestens einer Durchführungsöffnung; die flexible Schürze (14) hat dabei mindestens eine Tasche (37; 94; 94') und
mindestens ein röntgendichtes Teil (12), das in der mindestens einen Tasche (37) verfügbar und um die mindestens eine Durchführungsöffnung (30; 40) einstellbar ist;
wobei die mindestens eine Tasche (37 94; 94') so angeordnet ist, dass sie eine Öffnung (16) hat, die das Einsetzen des röntgendichten Teils (12) erlaubt, und einen Verschluss (18), wobei die Öffnung (16) über den Verschluss (18) in luft-/flüssigkeitsdichter Weise verschließbar ist.

2. Eine Strahlungsschutzanordnung (10) gemäß Anspruch 1, wobei die Öffnung (16) entlang eines Rands der Schürze angeordnet ist.

3. Die Strahlungsschutzanordnung (10) gemäß Anspruch 1, wobei die flexible Schürze (14) eine Vielzahl von Taschen (37 94; 94') und eine Vielzahl von röntgendichten Teilen (12) hat, die in den besagten Taschen (37 94; 94') untergebracht sind und wobei vorzugsweise die röntgendichten Teile (12) im Wesentlichen die mindestens eine Durchführungsöffnung (30; 40) umgeben.

4. Die Strahlungsschutzanordnung (10) gemäß Anspruch 1, wobei
(i) das mindestens eine röntgendichte Teil (12) eine Vielzahl von starren Abschnitten (56) einschließt, die zueinander faltbar sind oder
(ii) das mindestens eine röntgendichte Teil (12) plastisch verformbar ist.

5. Die Strahlungsschutzanordnung (10) gemäß Anspruch 4, die darüberhinaus eine Klebefläche aufweist, die die mindestens eine Durchführungsöffnung (30; 40) umgibt.

6. Die Strahlungsschutzanordnung (10) gemäß der in Anspruch 4 aufgeführten Option (i), die darüberhinaus flexible Verbindungen (56) einschließt, die die nebeneinander liegenden Abschnitte der besagten Vielzahl von starren Abschnitten miteinander verbinden, jede dieser flexiblen Verbindungen (56) hat dabei ein Stecker-Teil (58) und ein Buchsen-Teil (58').

7. Die Strahlungsschutzanordnung (10) gemäß der in Anspruch 4 aufgeführten Option (i), wobei das röntgendichte Teil einen Draht (46) einschließt, der durch die besagte Vielzahl der starren Abschnitte (56) verläuft, dieser Draht (46) ist in der Lage, die Vielzahl von starren Abschnitten (56) in einer gefalteten Konfiguration zu spannen, und vorzugsweise darüberhinaus einen Griff (44) einschließt, der an diesem Draht (46) befestigt ist.

8. Die Strahlungsschutzanordnung (10) gemäß Anspruch 1, wobei das mindestens eine röntgendichte Teil (12) eine ringförmige Wand mit einer Öffnung (38) hat, die diese Wand umlaufend unterbricht.

9. Die Strahlungsschutzanordnung (10) gemäß Anspruch 8, wobei die Schürze (14) so gestaltet ist, dass sie ähnlich geformt ist wie die ringförmige Wand des mindestens einen röntgendichten Teils (10) und darüberhinaus einen Materialstreifen (32) einschließt, der die Öffnung der umlaufend unterbrochenen Wand überbrückt.

10. Die Strahlungsschutzanordnung (10) gemäß Anspruch 9, wobei die Schürze (14) gegenüberliegende Seiten hat und darüberhinaus Haftnoppen-Material einschließt, das an mindestens einer der Seiten befestigt ist.

11. Die Strahlungsschutzanordnung (10) gemäß Anspruch 1, wobei das röntgendichte Teil (12) funktionsfähig mit einem starren Auflage-Teil verbunden ist, das starre Auflage-Teil (98; 99) hat dabei einen im Wesentlichen flachen Grundflächen-Teil (90; 98) und mindestens eine nach oben stehende Seitenwand (88; 94; 100) und optional weist die Seitenwand ein Paar aufrecht stehender Seitenwände (88) auf, wobei jede der Seitenwände von der Grundfläche (90; 98) abgenommen werden kann, oder der flache Grundflächen-Teil (90; 98) so angeordnet ist, dass er im Gebrauch unter dem Patienten hindurch geschoben wird und die aufrecht stehende Seitenwand einen gekrümmten Teil (100) aufweist.

12. Die Strahlungsschutzanordnung (10) gemäß Anspruch 11, wobei das röntgendichte Teil (94) frei von der Seitenwand (90: 98) herabhängt und abnehmbar an der Seitenwand (90: 98) über eine Vielzahl einzelner Verschlüsse (18) befestigt werden kann.

13. Ein Verfahren zur Bereitstellung einer sterilen Barriere gegenüber Strahlung in einem operativen Verfahren, das Folgendes aufweist:
das Auflegen einer flexiblen Schürze (14) mit einer Tasche (37 94; 94'), die so angeordnet ist, dass sie eine Öffnung (16) hat, die das Einsetzen von mindestens einem röntgendichten Teil (12) erlaubt, einen Verschluss (18), wobei die Öffnung (16) über einen Verschluss (18) in luft-/flüssigkeitsdichter Weise verschlossen werden kann, die Schürze hat dabei eine sterile Außenfläche und mindestens eine Durchführungsöffnung (30; 40) über einem Patienten; und
die Einsetzung des mindestens einen röntgendichten Teils (12) in die Tasche (37, 94; 94') um die mindestens eine Durchführungsöffnung (30; 40).

14. Das Verfahren gemäß Anspruch 13, das darüberhinaus die Bereitstellung der Schürze (14) mit einer Vielzahl von Taschen (37 94; 94') einschließt, die über eine Vielzahl von röntgendichten Teilen (12) in den Taschen (37 94; 94') verfügen.

15. Das Verfahren gemäß Anspruch 13, das darüberhinaus eines der folgenden Elemente einschließt:
(1) die plastische Verformung des röntgendichten Teils (12) nach dem Einsetzen des röntgendichten Teils in die Tasche (37 94; 94') oder
(2) das Einklappen des röntgendichten Teils (12) vor dem Einsetzen des röntgendichten Teils (12) in die Tasche (37 94; 94') und das Aufklappen des röntgendichten Teils (12), sobald das röntgendichte Teil in die Tasche (37 94; 94') eingesetzt ist.

## Revendications

1. Ensemble de protection contre les rayonnements (10), destiné à la protection de l'opérateur contre le rayonnement diffus, comprenant :
un champ souple (14) comportant une surface extérieure stérile et au moins une ouverture de passage ; le champ souple (14) comportant au moins une poche (37; 94; 94') et
au moins un élément radio-opaque (12) pouvant être placé dans la ou les poches (37) et agencé autour de ladite au moins une ouverture de passage (30; 40) ;
dans lequel la ou les poches (37 94; 94') sont agencées pour qu'une ouverture (16), qui permet l'insertion de l'élément radio-opaque (12) et un élément de fixation (18), dans lequel l'ouverture (16) est refermable par l'élément de fixation (18) d'une manière étanche à l'air / au fluide.

2. Un ensemble de protection contre les rayonnements (10) selon la revendication 1, dans lequel l'ouverture (16) est agencée le long d'un bord du champ.

3. L'ensemble de protection contre les rayonnements (10) de la revendication 1, dans lequel ledit champ souple (14) comporte une pluralité de poches (37 94; 94") et une pluralité d'éléments radio-opaques (12) placés dans lesdites poches (37 94; 94') et de préférence lesdits éléments radio-opaques (12) qui entourent pratiquement ladite au moins une ouverture de passage (30; 40).

4. L'ensemble de protection contre les rayonnements (10) de la revendication 1 dans lequel
(i) ledit au moins un élément radio-opaque (12) comprend une pluralité de sections rigides (56) pliables l'une par rapport à l'autre
(ii) ledit au moins un élément radio-opaque (12) est plastiquement déformable.

5. L'ensemble de protection contre les rayonnements (10) de la revendication 4 comprenant en outre une surface adhésive entourant ledit au moins une ouverture de passage (30; 40).

6. L'ensemble de protection contre les rayonnements (10) de l'option de la revendication 4 (i) comprenant en outre des articulations souples (56) se joignant de manière adjacente à celles de ladite pluralité de sections rigides l'une à l'autre, chacune des articulations souples (56) comportant un élément mâle (58) et un élément femelle (58').

7. L'ensemble de protection contre les rayonnements (10) de l'option de la revendication 4 (i), dans lequel ledit élément radio-opaque comprend un filament (46) en boucle à travers ladite pluralité de sections rigides (56), ledit filament (46) étant utilisable pour solliciter ladite pluralité de sections rigides (56) dans une configuration pliée et comprenant de préférence en outre une poignée (44) fixée à ce filament (46).

8. L'ensemble de protection contre les rayonnements (10) de la revendication 1, dans lequel ledit au moins un élément radio-opaque (12) comporte une paroi annulaire avec une ouverture (38) rendant ladite paroi circonférentiellement discontinue.

9. L'ensemble de protection contre les rayonnements (10) de la revendication 8, dans lequel ledit champ (14) est conçu en étant façonnée de façon similaire à ladite paroi annulaire dudit au moins un élément radio-opaque (10) et comportant en outre une bande de matériau (32) comblant ladite ouverture de ladite paroi circonférentiellement discontinue.

10. L'ensemble de protection contre les rayonnements (10) de la revendication 9, dans lequel ledit champ (14) comporte des faces opposées et comprenant en outre un matériau de préhension à friction fixé à au moins une desdites faces.

11. L'ensemble de protection contre les rayonnements (10) de la revendication 1, dans lequel l'élément radio-opaque (12) est connecté fonctionnellement à un élément de support rigide, l'élément de support rigide (98; 99) comportant une partie de base pratiquement plate (90; 98) et au moins une paroi latérale verticale (88; 94; 100) et, en option, la paroi latérale comporte une paire de parois latérales verticales (88) chacune des parois latérales étant détachable de la base (90; 98) ou la partie de base plate (90; 98) est agencée pour glisser sous le patient lors de l'utilisation et la paroi latérale verticale comprend une partie incurvée (100).

12. L'ensemble de protection contre les rayonnements (10) de la revendication 11, dans lequel l'élément radio-opaque (94) pend librement de la paroi latérale (90 : 98) et peut être fixé de façon détachable de la paroi latérale (90: 98) par une pluralité d'éléments de fixation discrets (18).

13. Un procédé de fourniture d'une barrière stérile contre les rayonnements dans une procédure chirurgicale, comprenant :
la pose d'un champ souple (14) comportant une poche (37 94; 94') agencée pour comporter une ouverture (16) qui permet l'insertion d'au moins un élément radio-opaque (12) et un élément de fixation (18), dans lequel l'ouverture (16) peur être fermée par l'élément de fixation (18) d'une manière étanche à l'air / au fluide, le champ comportant une surface extérieure stérile et au moins une ouverture de passage (30; 40) au-dessus d'un patient ; et
le placement du ou des éléments radio-opaques (12) dans la poche (37 94; 94') autour de la ou des ouvertures de passage (30; 40).

14. Le procédé de la revendication 13 comprenant en outre la fourniture du champ (14) avec une pluralité de poches (37 94; 94') le placement d'une pluralité d'éléments radio-opaques (12) dans les poches (37 94; 94').

15. Le procédé de la revendication 13, comprenant en outre :
(1) la déformation de façon plastique de l'élément radio-opaque (12) après avoir placé les éléments radio-opaques dans la poche (37 94; 94') ou
(2) le pliage des éléments radio-opaques (12) avant de placer les éléments radio-opaques (12) dans la poche (37 94; 94') et le dépliage de l'élément radio-opaque (12) après avoir placé les éléments radio-opaques dans la poche (37 94; 94').
